# EUROPEAN PATENT SPECIFICATION

(11) **EP 3 958 835 B1**
(45) Date of publication and mention of the grant of the patent: **24.05.2023**
(21) Application number: 20720452.0
(22) Date of filing: 24.04.2020
(51) Int. Cl.: A61K 47/14, A61K 47/26, A61P 17/14, A61K 9/00, A61K 9/10, A61K 9/19

(54) **TOPICAL FORMULATION**
TOPISCHE FORMULIERUNG
FORMULATION TOPIQUE

(30) Priority: 24.04.2019 EP 19170868
(43) Date of publication of application: 02.03.2022
(73) Proprietor: Follicum AB, 223 63 Lund (SE)
(72) Inventor: LINDAHL, Åke, 217 75 Malmö (SE); ALENFALL, Jan, 234 37 Lomma (SE); EKBLAD, Maria, 247 32 Södra Sandby (SE)
(74) Representative: Høiberg P/S
(86) International application number: PCT/EP2020/061453
(87) International publication number: WO 2020/216896

(56) References cited:
- EP-A1- 2 305 292
- EP-A1- 3 087 999
- WO-A1-2018/202870

## Description

### Technical field

The present invention relates to a composition for topical delivery.

### Background

Drug molecules larger than 600 Da normally have difficulties in permeating the skin. For polypeptides and peptides, the problem is accentuated even if the molecular weight is below 600 Da, since amino acids in general are too polar to penetrate the stratum corneum in an appropriate way, which is why most peptide- or protein-based drug are administered by parenteral formulations, which is undesirable for many purposes including targeting of topical, e.g. dermal disorders and diseases.

The polarity of many peptides is thus also problematic when attempting administration to hair follicles, e.g. for treating disorders or diseases associated with the hair follicle.

For a peptide to be biologically active, and prepared for parenteral administration, it typically needs to be dissolved in an aqueous solution, which often results in severe stability problems with undesired properties like decreased shelf life and loss of biological activity as effects.

Therefore, there is a need for drug formulations capable of overcoming these delivery and stability problems.

### Summary

Topical delivery of active ingredients through the skin or other body surfaces for both local and systemic effects is attractive to patients for a variety of reasons. For local treatment, a lower dose is required creating a less bioburden. The initial toxicological experiments are less costly and time consuming and the amount of drug required is normally less. Another advantage of topical delivery is that the concentration in the target tissue is less variable by achieving a depot of the drug. In addition to convenience, topical formulations avoid the irritation of gastrointestinal tract that often accompany pills and capsules.

The present inventors have developed a topical formulation that promotes penetration of large molecular weight molecules, and at the same time keeps the drug in the solid state to provide chemical stability during the shelf life of the product. Importantly, the topical formulation is suitable for delivery of large molecular weight molecules into follicles and associated tissue.

Sebum, which is the main component of the hair follicle, is a mixture of lipids. The present inventors have developed a drug formulation with biophysical properties enabling solution in and passage through the sebum, in quantities sufficient for biological response.

The follicular administration route presents an improvement over invasive administration forms. When a drug is injected by subcutaneous or intradermal routes, the drug concentration at administration site decreases instantly, and within a few hours the local drug concentration is not sufficient for generation of a biological effect. When treatment of local sites of the disease is desired, parenteral administration is undesirable. Generally, a constant drug concentration during the treatment period is more favourable. Follicular delivery is one non-invasive way of overcoming this problem. In addition, follicular delivery allows for a depo effect as the drug load can be maximized.

Hydrophilic particles containing the active agent, such as a peptide, and a saccharide dispersed in a lipid based vehicle generate drug penetration into the follicle and its surroundings. This way of presenting the active agent to the disease area is useful for the treatment of many disorders and diseases, including e.g. hair loss, hirsutism, psoriasis, atopic dermatitis, eczema, precarcinogenic states and skin infections.

In one aspect, the present invention provides a composition comprising:
a) a peptide;
b) a saccharide ; and
c) a lipid.

In one aspect, the present invention relates to a composition comprising:
a. 0.01 to 2 wt% peptide or peptide derivative;
b. 0.01 to 4 wt% saccharide or modified saccharide;
c. 35 to 50 wt% petrolatum; and
d. 40 to 60 wt% isopropyl myristate,
with the proviso that the sum of the components of the total composition does not exceed 100 wt%.

In one aspect, the composition is for use as a medicament.

In one aspect, the composition is for use in the treatment of dermatological conditions.

In another aspect, the present invention provides the use of a compound according to the above aspect for stimulating hair growth in a mammal, wherein the use is cosmetic.

In yet another aspect, the present invention provides the use of a compound according to the above aspects for the treatment or prevention of baldness.

In one aspect, the present invention provides a method for stimulating hair growth, said method comprising administering topically to a patient in need thereof, an effective amount of the composition of any one of the above aspects.

In another aspect, the present invention provides a method for treating hair loss, said method comprising administering topically to a patient in need thereof, an effective amount of the composition of any one of the above aspects.

In one aspect, the present invention provides a method for topical delivery of a composition according to the above aspects to a patient in need thereof, comprising applying an effective amount of the composition of the above aspects to a topical application site of the patient.

In another aspect, the present invention provides a method of manufacturing a medicament for topical delivery of a composition according to any one of the above aspects, comprising the following steps:
a) mixing the peptide or peptide derivative with a saccharide or modified saccharide;
b) freeze drying the mixture of a);
c) mixing b) with a lipid and a surfactant;
d) grinding the mixture of c); and
e) optionally mixing the mixture of d) with a lipid and a thickener.

### Description of Drawings

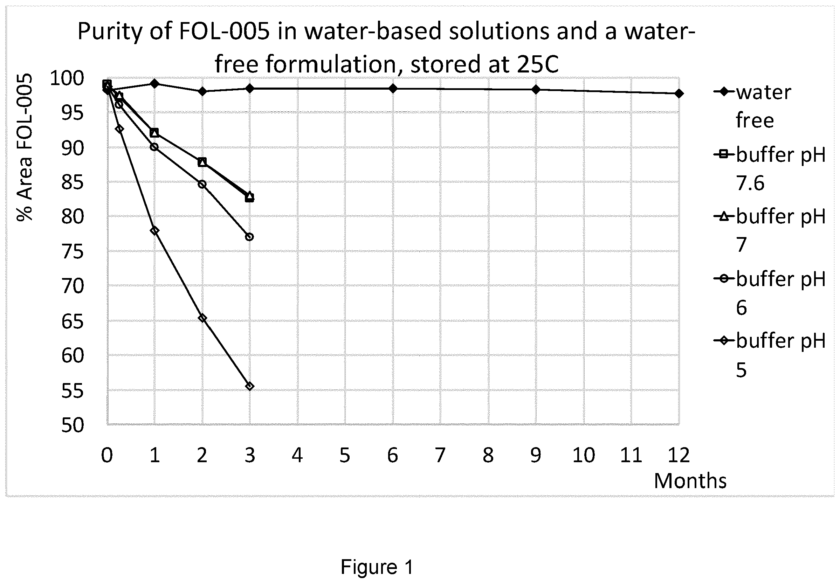

**Figure 1****. Stability study** - **comparison of water-based solutions of FOL-005 and an essentially water free formulation**
   FOL-005 lyophilized powder was dissolved in phosphate/citrate buffer of pHs 5, 6, 7 or 7.6. Samples of each formulation were analysed for purity using high-pressure liquid chromatography after up to 3 months of storage at 25°C respectively. In addition, an essentially water-free formulation of FOL-005 was stored at 25°C and samples were analysed for purity after up to 12 months of storage.

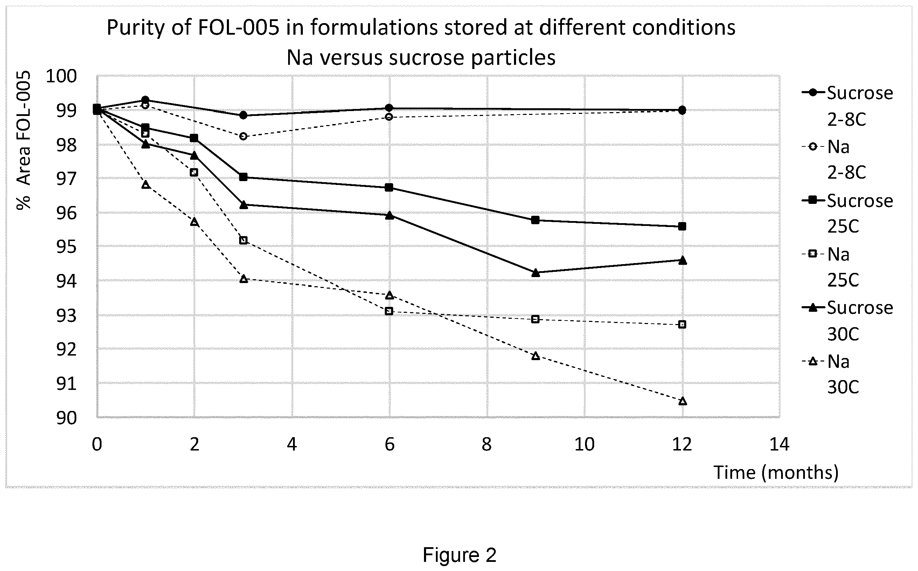

**Figure 2****. Stability study** - **comparison of Na-FOL--005 and Na-FOL-005-Sucros**
   FOL-005/sucrose lyophilized powder was formulated to a suspension in dried or un-dried isopropyl myristate, paraffin oil/petrolatum, Sorbitan laurate and glyceryl behenate. Samples of each formulation were analysed for purity using high pressure liquid chromatography after up to 12 months of storage at 2-8, 25 and 30°C respectively.

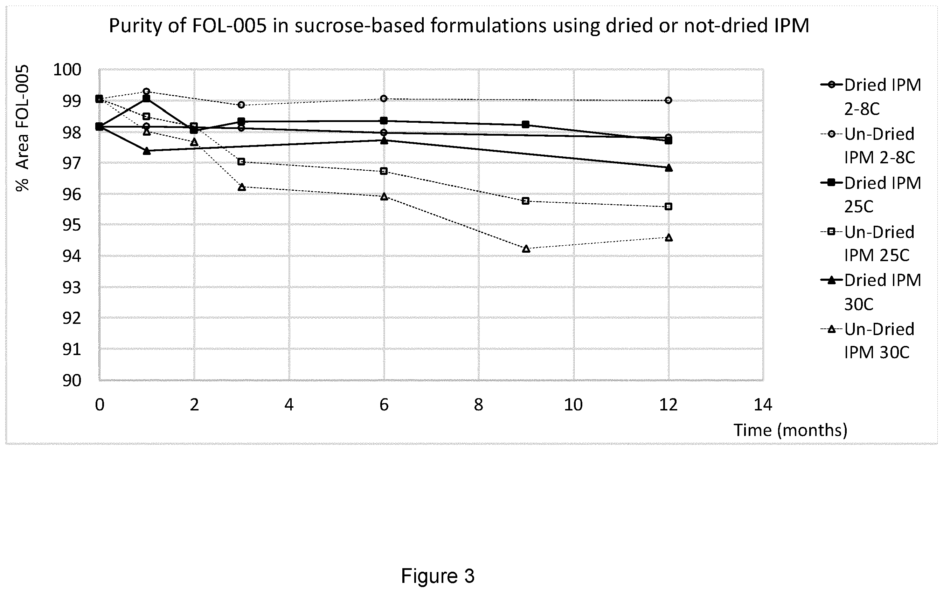

**Figure 3****. Stability study** - **comparison of dried IPM and usual IPM**
   FOL-005/sucrose lyophilized powder was formulated to a suspension in dried or un-dried isopropyl myristate, paraffin oil/petrolatum, Sorbitan laurate and glyceryl behenate. Samples of each formulation were analysed for purity using high pressure liquid chromatography after up to 12 months of storage at 2-8, 25 and 30°C respectively.

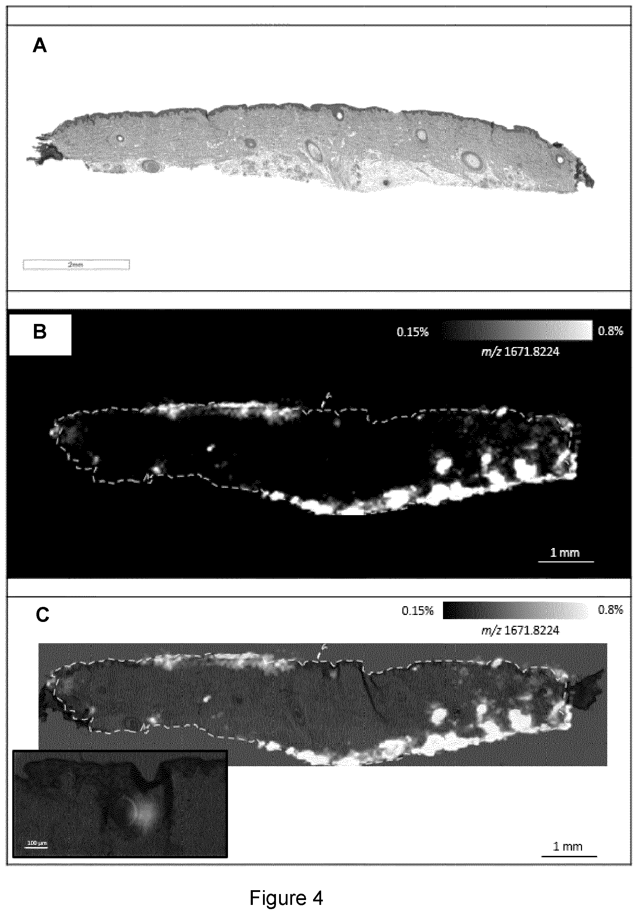

**Figure 4****: *Ex vivo* study**
   FOL-005 distribution in pig inner ear skin section treated with FOL-005/sucrose formulation with isopropyl myristate, petrolatum, Sorbitan laurate and glyceryl behenate. The formulation was applied to the skin sample at 0 and 24 h. At 48 h the sample was frozen and subsequently sectioned and analysed using MALDI MSI. The concentration of FOL-005 is displayed in black-grey-white. White areas contain high concentration of FOL-005 and black areas contain low concentrations. A) H & E Staining (Adjacent Tissue Section), B) FOL-005 MSI Distribution in the treated tissue section, C) Overlay between the MSI Distribution and the H & E staining.

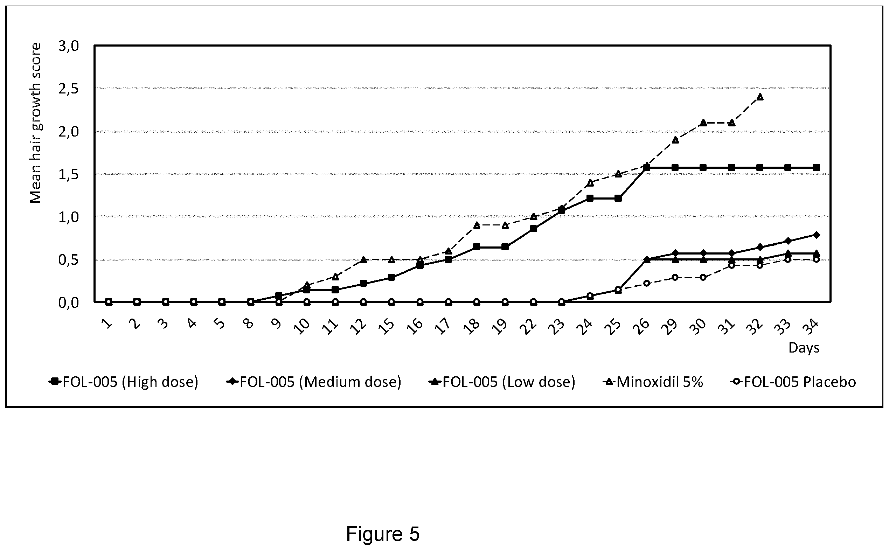

**Figure 5****: *In vivo* efficacy study**
   C57BI6 mice in stable telogen phase (age 6-9 weeks) were carefully clipped. Topical formulation of FOL-005 at three different strengths and placebo was applied at the clipped area once daily, 5 days per week until day 26. For comparison Minoxidil was administered to one group twice daily, 5 days per week until day 26. The hair growth was scored according to an established system, from 0 (no hair growth, pink skin) to 3 (dense, normal coat hair)

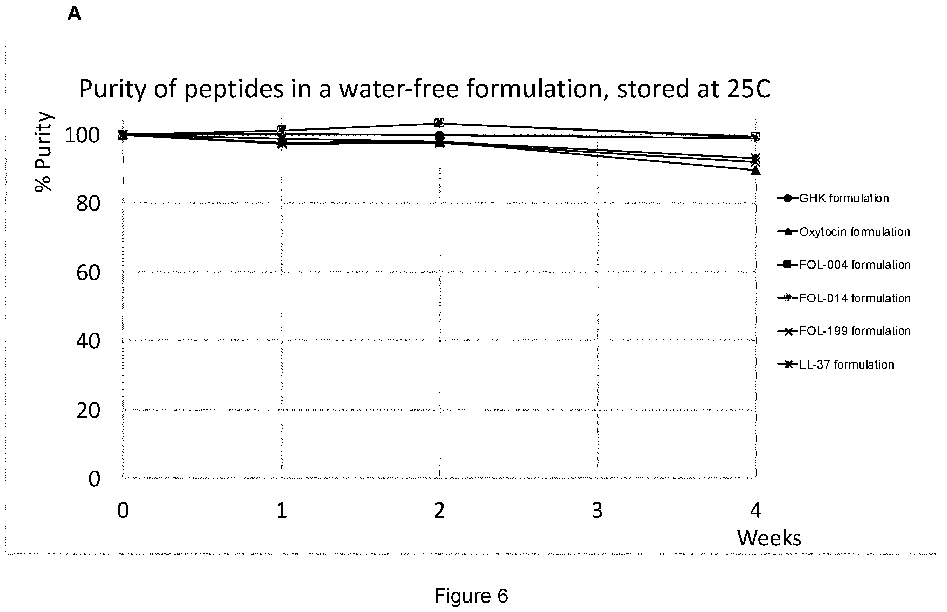

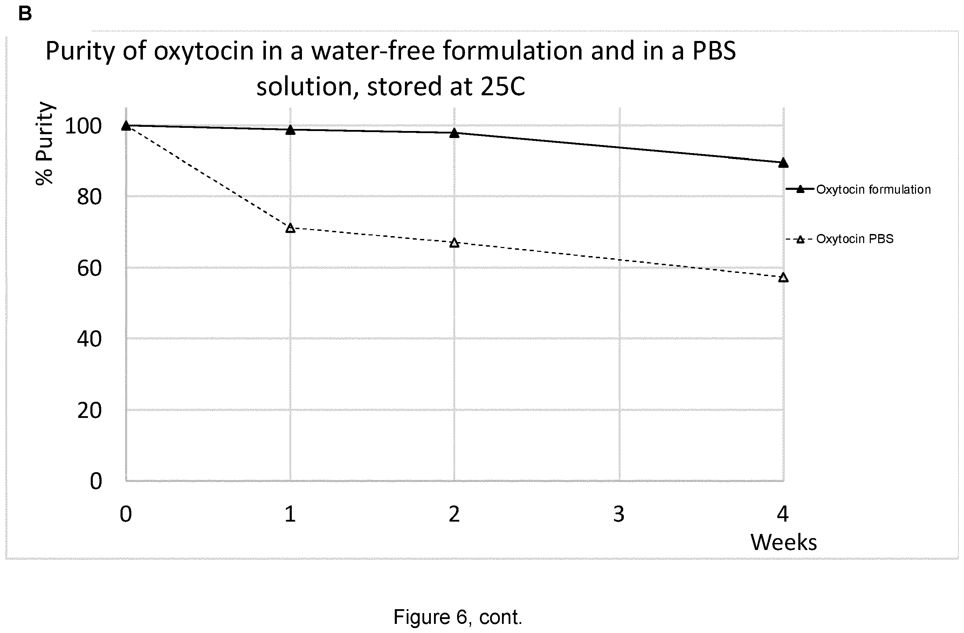

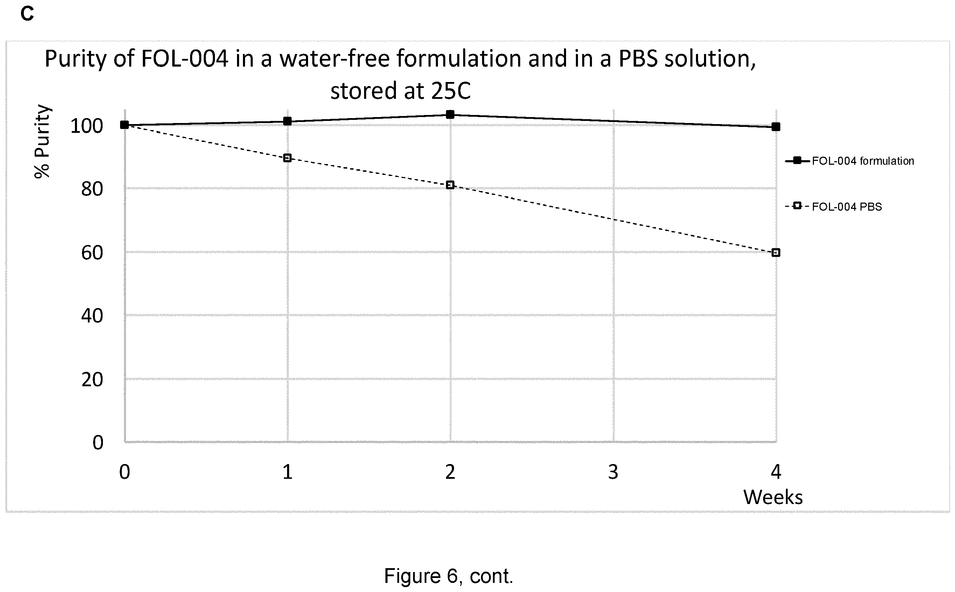

**Figure 6****: Compositions comprising peptides**
   **A)** Stability of peptides in a water-free formulation (see Example 8). Water-free formulations of six peptides of different size and amino acid sequence were prepared. The concentration of the peptides was 0.2% and the following excipients and concentrations (w/w %) were used: Sucrose 0.4%, Span20 4%, Glyceryl behenate 3%, Isopropyl myristate 50% and petrolatum 42.4%. The formulations were stored at 20 +/-5°C and the peak purity was analysed using high pressure liquid chromatography at study start and after 1, 2 and 4 weeks of storage. **B)** Stability of oxytocin in a water-free formulation and in a PBS solution (see Example 8). A water-free formulation of oxytocin was prepared. The following excipients and concentrations (w/w %) were used: Oxytocin 0.2%, Sucrose 0.4%, Span20 4%, Glyceryl behenate 3%, Isopropyl myristate 50% and petrolatum 42.4%. In addition, a 0.2% solution of Oxytocin in PBS, pH 7.4 was prepared. The formulation and the solution were stored at 20 +/- 5°C and the peak purity was analysed using high pressure liquid chromatography at study start and after 1, 2 and 4 weeks of storage. **C)** Stability of FOL-004 in a water-free formulation and in a PBS solution (see Example 8). A water-free formulation of FOL-004 was prepared. The following excipients and concentrations (w/w %) were used: FOL-004 0.2%, Sucrose 0.4%, Span20 4%, Glyceryl behenate 3%, Isopropyl myristate 50% and petrolatum 42.4%. In addition, a 0.2% solution of FOL-004 in PBS, pH 7.4 was prepared. The formulation and the solution were stored at 20 +/- 5°C and the peak purity was analysed using high pressure liquid chromatography at study start and after 1, 2 and 4 weeks of storage.

### Detailed description

In one aspect, the present invention provides a composition comprising:
a) a peptide;
b) a saccharide ; and
c) a lipid.

In one aspect, the present invention relates to a composition comprising:
a. 0.01 to 2 wt% peptide or peptide derivative;
b. 0.01 to 4 wt% saccharide or modified saccharide;
c. 35 to 50 wt% petrolatum; and
d. 40 to 60 wt% isopropyl myristate,
with the proviso that the sum of the components of the total composition does not exceed 100 wt%.

### The peptide

The composition of the present invention comprises at least one peptide or peptide derivative. In one embodiment, the peptide or peptide derivative is the active agent. Importantly, the present composition promotes penetration of the peptide or peptide derivative, and at the same time keeps the peptide or peptide derivative in the solid state to provide chemical stability during the shelf life of the product.

The term 'peptide' as used herein refers to a molecule comprising two or more amino acid residues joined to each other by peptide bonds. The terms "peptide" and "polypeptide" are used herein interchangeably throughout. In one embodiment, the term 'peptide' also includes peptide derivatives.

The term 'peptide derivative' as used herein relates to a peptide which is modified or derivatized. In one embodiment, the peptide derivative is a peptide as disclosed herein which is conjugated to a moiety, such as a moiety selected from the group consisting of polyethylene glycol (PEG), monosaccharides, fluorophores, chromophores, radioactive compounds, and cell-penetrating peptides. In one embodiment, the peptide is glycosylated.

In one embodiment, the peptide derivative is a peptide as disclosed herein which is modified by being glycosylated or by PEGylation, amidation, esterification, acylation, acetylation and/or alkylation.

In one embodiment, the peptide derivative is a peptide as disclosed herein which is fused to another polypeptide, such as a polypeptide selected from the group consisting of glutathione-S-transferase (GST) and protein A, or to a tag.

The term 'amino acid' as used herein includes the standard twenty genetically-encoded amino acids and their corresponding stereoisomers in the 'D' form (as compared to the natural 'L' form), omega-amino acids and other naturally-occurring amino acids, unconventional amino acids (e.g., α,α-disubstituted amino acids, N-alkyl amino acids, *etc*.) and chemically derivatised amino acids (see below).

When an amino acid is being specifically enumerated, such as `alanine' or `Ala' or `A', the term refers to both L-alanine and D-alanine unless explicitly stated otherwise. Other unconventional amino acids may also be suitable components for peptides of the present invention, as long as the desired functional property is retained by the peptide. For the peptides shown, each encoded amino acid residue, where appropriate, is represented by a single letter designation, corresponding to the trivial name of the conventional amino acid.

In accordance with convention, the amino acid sequences disclosed herein are provided in the N-terminus to C-terminus direction.

In one embodiment, the peptide comprises or consists of from 3 to 50 amino acid residues, such as from 3 to 40 amino acid residues, such as 5 to 30 amino acid residues, such as 10 to 25 amino acid residues.

In one embodiment, the peptide consists of less than 500 amino acids, for example less than 400, 350, 340, 330, 320, 310, 300, 290, 280, 270, 260, 250, 200, 150, 100, 50, 40, 30, 20, 15, 10 or fewer amino acids in length. In one embodiment, the peptide consists of at least 3 amino acids, such as at least 5, such as at least 10, such as at least 15, such as at least 20, such as at least 25, such as at least 30 or more amino acids in length.

In one embodiment, the peptide comprises or consists of tandem repeats. In one embodiment, the peptide is cyclic.

In one embodiment, the peptide is amphipathic. An amphiphile is a chemical compound possessing both hydrophilic (water-loving, polar) and lipophilic (fat-loving) properties. Such a compound is called amphiphilic or amphipathic.

In one embodiment, the peptide or peptide derivative is an antimicrobial peptide. In one embodiment, the antimicrobial peptide is selected from the group consisting of polymyxin B, LL37 (SEQ ID NO: 188), and FOL-199 (SEQ ID NO: 185).

In one embodiment, the peptide or peptide derivative is an anti-inflammatory peptide. In one embodiment, the anti-inflammatory peptide is selected from the group consisting of FOL-005 (SEQ ID NO: 1), FOL-004 (SEQ ID NO: 69) and FOL-199 (SEQ ID NO: 185).

In one embodiment, the active component of the compositions of the invention are derived from a naturally-occurring peptide such as an osteopontin protein (e.g. the peptide comprises an amino acid sequence corresponding to that of a modified, for example mutated, version of a naturally-occurring osteopontin protein). A characterising feature of the osteopontin-derived peptide in the compositions of the invention is that the RGD domain naturally present in osteopontin is inactivated such that it is non-functional (at least in part). For example, inactivation of the RGD domain may prevent the osteopontin-derived peptide from binding to one or more of the integrins which bind the naturally occurring osteopontin protein.

Thus, by "modified osteopontin peptide" we include peptides corresponding to a modified form of a naturally-occurring osteopontin protein in which the RGD domain is non-functional (at least in part), as well as fragments and variants thereof which retain a hair-stimulatory property of the 'full length' modified osteopontin.

In one embodiment, the peptide may comprise or consist of a fragment of the amino acid sequence of SEQ ID NO: 1 FOL-005, or a variant thereof.

The term "fragment" as used herein may include any fragment, preferably a biologically active fragment of an amino acid sequence described herein. In one embodiment, the fragment is of at least 6 contiguous amino acids of the amino acid sequence of SEQ ID NO: 1, for example at least 7, 8, 9, 10, 11, 12, 13, 14, 15, 16, 17, 18, 19, 20, 30, 40, 50, 100, 150, 200, 210, 220, 230, 240, 250, 255, 260, 265, 270, 275, 280, 285, 286, 287, 288, 289 290, 291 or 292 contiguous amino acids of SEQ ID NO: 1.

By "variant" we mean that the peptide does not share 100% amino acid sequence identity with SEQ ID NO: 1, *i.e.* one or more amino acids of SEQ ID NO: 1 must be mutated. For example, the peptide may comprise or consist of an amino acid sequence with at least 50% identity to the amino acid sequence of SEQ ID NO: 1, more preferably at least 60%, 70% or 80% or 85% or 90% identity to said sequence, and most preferably at least 95%, 96%, 97%, 98% or 99% identity to said amino acid sequence.

The variant peptide may also comprise one or more additional amino acids, inserted at the N- and/or C-terminus and/or internally within the amino acid sequence of SEQ ID NO: 1. For example, the peptide may comprise or consist of at least 2, 3, 4, 5, 6, 7, 8, 9, 10, 15 or 20 additional amino acids at the N- and/or C-terminus and/or internally.

Advantageously, the variant peptide is non-naturally occurring.

In one embodiment, a solid material of FOL-005 was used to achieve a chemically stable formulation of FOL-005 and to direct FOL-005 to the skin via the hair follicles, for this FOL-005 was milled to small particles with the major fraction below betwen 1µm and 5µm in diameter.

In one embodiment, the peptide shares amino acid sequence similarity with a subregion of naturally occurring tenascin proteins. In some embodiments, said peptide may be regarded as an active fragment of a naturally-occurring tenascin protein or a variant of such as a fragment.

In one embodiment, the peptide comprises or consists of:
i) an amino acid sequence of the general formula:
   KX₂LAX₅X₆X₇X₈IX₁₀LX₁₂YGIK **(SEQ ID NO: 140)**
   wherein:
   X₂ is C, P or G;
   X₅ is E or G;
   X₆ is C, D or I;
   X₇ is D, I, S or G;
   X₈ is S, D or G;
   X₁₀ is E or G;
   X₁₂ is S or T;

   with the proviso that if X₁₂ is T, the peptide comprises no more than 25 amino acid residues; and
   with the proviso that if X₂ is P, X₅ is E, X₆ is I, X₇ is D, X₈ is S, X₁₀ is E and X₁₂ is S, the peptide comprises no more than 85 amino acid residues;
ii) an amino acid sequence of the general formula:
   X₁₀LX₁₂YGIK **(SEQ ID NO: 177)**
   wherein:
   X₁₀ is E or G;
   X₁₂ is S or T;

   with the proviso that if X₁₂ is T, the peptide comprises no more than 25 amino acids; and
   with the proviso that if X₁₀ is E and X₁₂ is S, the peptide comprises no more than 85 amino acid residues;
iii) an amino acid sequence of the general formula:
   VDVPZ₅GDISLAYZ₁₃LR **(SEQ ID NO: 164)**
   wherein:
   Z₅ is E or N;
   Z₁₃ is R or G;
iv) an amino acid sequence of the general formula: VDTYDGZ₇Z₈SVVYGLR **(SEQ ID NO: 165)**
   wherein:
   Z₇ is D or G;
   Z₈ is I or G;
v) an amino acid sequence of the general formula:
   GDPNZ₅Z₆Z₇Z₈Z₉SVVYGLR **(SEQ ID NO: 166)**
   wherein:
   Z₅ is D or G;
   Z₆ is D or G
   Z₇ is I or R;
   Z₈ is G or absent;
   Z₉ is D or absent;
vi) an amino acid sequence of the general formula:
   KX₂LAX₅X₆X₇X₈IX₁₀LSYGIK **(SEQ ID NO: 162)**
   wherein:
   X₂ is C, P or G;
   X₅ is E or G;
   X₆ is C, I or absent;
   X₇ is D, G or absent;
   X₈ is S, G or absent;
   X₁₀ is E or G;
vii) an amino acid sequence of the general formula:
   KX₂LAX₅IX₁₀LSYGIK **(SEQ ID NO: 163)**
   wherein:
   X₂ is C, P or G;
   X₅ is E or G;
   X₁₀ is E or G;
viii) an amino acid sequence of the general formula:
   Z₇Z₈SZ₁₀Z₁₁YGLR **(SEQ ID NO: 178)**
   wherein:
   Z₇ is D or G;
   Z₈ is I or G;
   Z₁₀ is V or L;
   Z₁₁ is V or A;
   or
ix) an amino acid sequence of the general formula:
   VDZ₃Z₄Z₅GZ₇Z₈SZ₁₀Z₁₁YGLR **(SEQ ID NO: 68)**
   wherein:
   Z₃ is T or V;
   Z₄ is Y or P;
   Z₅ is D or N;
   Z₇ is D or G;
   Z₈ is I or G;
   Z₁₀ is V or L;
   Z₁₁ is V or A.

The term 'absent' as used herein, e.g. "X₆ is C, I or absent" is to be understood as that the amino acid residues directly adjacent to the absent amino acid are directly linked to each other by a conventional amide bond.

In one embodiment, the peptide comprises or consists of an amino acid sequence selected from the group consisting of GDPNDGRGDSVVYGLR (SEQ ID NO: 137), VDTYDGGISVVYGLR (SEQ ID NO: 138), and VDTYDGDGSVVYGLR (SEQ ID NO: 139). VDVPEGDISLAYGLR (SEQ ID NO: 157), LDGLVRAYDNISPVG (SEQ ID NO: 158), GDPNGDISVVYGLR (SEQ ID NO: 159), VDVPNGDISLAYRLR (SEQ ID NO: 160) VDVPEGDISLAYRLR (SEQ ID NO: 161).

In one embodiment, the peptide comprises or consists of an amino acid sequence selected from the group consisting of GDPNDGRGDSVVYGLR (SEQ ID NO: 137), VDTYDGGISVVYGLR (SEQ ID NO: 138), and VDTYDGDGSVVYGLR (SEQ ID NO: 139). VDVPEGDISLAYGLR (SEQ ID NO: 157), LDGLVRAYDNISPVG (SEQ ID NO: 158), GDPNGDISVVYGLR (SEQ ID NO: 159), VDVPNGDISLAYRLR (SEQ ID NO: 160) VDVPEGDISLAYRLR (SEQ ID NO: 161), V(beta-D)TYDGDISVVYGLR (SEQ ID NO:167), VDTY(beta-D)GDISVVYGLR (SEQ ID NO: 168), VDTYDG(beta-D)ISVVYGLR (SEQ ID NO:169).

In one embodiment, the peptide comprise or consists of the amino acid sequence of VDTYDGDISVVYGLR (SEQ ID NO: 1; FOL-005) or a fragment/variant thereof.

In one embodiment, the peptide comprises or consists of the amino acid sequence of KPLAEIDSIELSYGIK (SEQ ID NO: 136, FOL-014) or a fragment/variant thereof.

In one embodiment, the peptide comprises or consists of the amino acid sequence of VDVPNGDISLAYGLR (SEQ ID NO: 69, FOL-004) or a fragment/variant thereof.

In one embodiment, the peptide comprises or consists of an amino acid sequence selected from the group consisting of KCLAECDSIELSYGIK (SEQ ID NO: 141), CLAEIDSC (SEQ ID NO: 142), CFKPLAEIDSIECSYGIK (SEQ ID NO: 143), KPLAEDISIELSYGIK (SEQ ID NO: 145), KPLAEIGDIELSYGIK (SEQ ID NO: 146), KPLAEGDIELSYGIK (SEQ ID NO: 147), KPLAEIELSYGIK (SEQ ID NO: 148), KPLAEIDSIELTYGIK (SEQ ID NO: 149), KPLAEIDGIELSYGIK (SEQ ID NO: 150), KPLAEIDGIELTYGIK (SEQ ID NO: 151), KPLAEIGSIELSYGIK (SEQ ID NO: 152), KGLAEIDSIELSYGIK (SEQ ID NO: 153), KPLAGIDSIGLSYGIK (SEQ ID NO: 154), KCLAEIDSCELSYGIK (SEQ ID NO: 155) and CFKPLAEIDSIEC (SEQ ID NO: 156), or a variant or fragment thereof.

In one embodiment, the peptide comprises or consists of an amino acid sequence selected from the group consisting of LAEIDSIELSYGIK (SEQ ID NO: 170), AEIDSIELSYGIK (SEQ ID NO: 171), EIDSIELSYGIK (SEQ ID NO: 172), IDSIELSYGIK (SEQ ID NO: 173), DSIELSYGIK (SEQ ID NO: 174), SIELSYGIK (SEQ ID NO: 175), IELSYGIK (SEQ ID NO: 176), or a variant or fragment thereof.

In one embodiment, the peptide comprises or consists of an amino acid sequence selected from the group consisting of KPLAEIDSIELSYGI (SEQ ID NO: 179), KPLAEIDSIELSYG (SEQ ID NO: 180), KPLAEIDSIELSY (SEQ ID NO: 181), KPLAEIDSIELS (SEQ ID NO: 182), KPLAEIDSIEL (SEQ ID NO: 183), KPLAEIDSIE (SEQ ID NO: 184), or a variant of fragment thereof.

In another embodiment, the peptide is selected from the group consisting of SEQ ID NO: 1, 136, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 67, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 135, 137, 138, 139, 141, 142, 143, 144, 145, 146, 147, 148, 149, 150, 151, 152, 153, 154, 155, 156, 157, 158, 159, 160, 161, 167, 168, 169, 170, 171, 172, 173, 174, 175, 176, 177, 178, 179, 180, 181, 182, 183, 184, 185, 186, 187, and 188.

In one embodiment, the peptide is selected form the group consisting of GHK (SEQ ID NO: 188), oxytocin (SEQ ID NO: 186), polymyxin B, LL37 (SEQ ID NO: 187), FOL-199 (SEQ ID NO: 185) and becaplermin.

### The saccharide

The composition comprises at least one saccharide or modified saccharide. Saccharides are made up of n monosaccharide units linked to each other by a glycosidic bond, wherein n is an integer. In one embodiment, the saccharide is selected from mono-, di- and trisaccharides. For monosaccharides, n is 1, for disaccharides, n is 2, and for trisaccharides, n is 3. In one embodiment, the saccharide is essentially consisting of a mono-, di- or trisaccharide, such as the properties of the saccharide are essentially determined by the mono-, di- or trisaccharide moiety. In one embodiment, the saccharide is a saccharide derivative or a modified saccharide, such as a sugar alcohol.

The melting point of a substance is the temperature at which it changes state from solid to liquid. At the melting point the solid and liquid phase exist in equilibrium. In one embodiment, the saccharide has a melting temperature between 60 and 140°C. In one embodiment, the saccharide has a melting temperature between 60 and 65°C, such as between 65 and 70°C, for example between 70 and 75°C, such as between 75 and 80°C, for example between 80 and 85°C, for example between 85 and 90°C, such as between 90 and 95°C, for example between 95 and 100°C, such as between 100 and 105°C, for example between 105 and 110°C, such as between 110 and 115°C, for example between 115 and 120°C, such as between 120 and 125°C, for example between 125 and 130°C, such as between 130 and 135°C, for example between 135 and 140°C.

In one embodiment, the saccharide is sucrose. In one embodiment, the saccharide is maltose. In one embodiment, the saccharide is trehalose. In one embodiment, the saccharide is raffinose. In on embodiment, the saccharide is maltotriose. In one embodiment, the saccharide is stachyose. In one embodiment, the saccharide is glucose. In one embodiment, the saccharide is dextran. In one embodiment, the saccharide is a sugar alcohol, such as mannitol.

In one embodiment, the weight ratio of saccharide and peptide of the composition is 1:9 to 9:1, such as 1:9 or 9:1. In one embodiment, the weight ratio of saccharide and peptide of the composition is 5:1 to 1:5, such as 2:1 or 1:1.

### Particles

Freeze-drying of the peptide together with sucrose may be performed to increase the dissolution of the peptide particles and to increase the chemical stability of the peptide. In one embodiment, the peptide and the saccharide form particles, which are hydrophilic. The particles may be in a solid, glass or rubber state. The particles can be manufactured by standard techniques such as freeze drying, spray drying or freeze spraying, followed by operations to reduce particle size to submicron levels.

In one embodiment, at least 50% of the particles have an average particle diameter of between 0.1 and 10 µm , such as between 0.1 and 1 µm, such as between 1 and 5 µm, such as between 5 and 10 µm, for example between 10 and 15 µm, such as between 15 and 20 µm, for example between 20 and 25 µm, such as between 25 and 30 µm, such as between 30 and 35 µm, for example between 35 and 40 µm, such as between 40 and 45 µm, for example between 45 and 50 µm.

In one embodiment, at least 50% of the particles have an average particle diameter of between 0.1 and 50 µm, for example between 0.1 and 15 µm, such as between 0.1 and 10 µm, such as between 0.1 and 2 µm.

In one embodiment, the size of the particles is below 50 µm, for example below 40 µm, such as below 30 µm, for example below 20 µm, such as below 10 µm.

In one embodiment, the composition comprises 0.01 to 10 wt% particles, such as 0.1 to 5 wt% particles, such as 0.1 to 2 wt% particles.

In one embodiment, the particles dissolve rapidly once coming in contact with water. Typically, the particles should dissolve within a minute in water at 37 °C.

### The lipid vehicle

The composition of the present invention comprises at least one lipid. The lipid function as a vehicle in the composition, i.e. serving as a medium for conveying the active ingredient. In one embodiment, the lipid vehicle comprises one or more different lipids. In one embodiment, the composition comprises one type of lipid. In one embodiment, the composition comprises two types of lipids.

Non-limiting examples of suitable lipids are mono-, di- and tri-esters of fatty acids, C6 to C22, and alcohols such as propanol, butanol, propyleneglycol and glycerol, and mixtures of lipids such as white or yellow soft paraffin. In one embodiment, the lipid is isopropyl myristate or isopropyl palmitate. In one embodiment, the lipid is selected from the group consisting of petrolatum, isopropyl myristate and glyceryl behenate.

The term 'petrolatum' as used herein refers to a semi-solid mixture of hydrocarbons (CAS number 8009-03-8). Petrolatum is also known as 'Petroleum jelly', 'white petrolatum', and 'soft paraffin', or multi-hydrocarbon. Petrolatum is also sold as Vaselin ^{®}.

In one embodiment, the lipid is a vaseline or a paraffin, such as paraffin oil. Paraffin oil or liquid paraffin oil is obtained in the process of petroleum distillation. Thus, the petroleum may be paraffin oil.

In one embodiment, isopropyl myristate is mixed with petrolatum 1:1. In one embodiment, the composition comprises isopropyl myristate and petrolatum. In one embodiment, the composition comprises isopropyl myristate and petrolatum in the weight ratio of 2:1 to 1:2, such as 3:2 to 2:3, such as about 1:1.

In one embodiment, the composition comprises petrolatum, isopropyl myristate and glyceryl behenate.

In one embodiment, the lipid is dried. In one embodiment, the isopropyl myristate is dried.

In one embodiment, the lipid is used to facilitate the distribution to hair follicles, i.e. a lipid vehicle that is compatible with the sebum content in the hair follicle and to increase the chemical stability of the peptide. Thus, in one embodiment, the lipid solubilizes sebum.

Upon distribution to hair follicles, the lipid vehicle is important as a carrier of the particles and to make contact with the content of the follicle. Examples of components of such vehicle are fluid (at room and body temperature) lipids that dissolve sebum. The amount of solid material in the suspension may vary depending on the dose required and on the size of the area to be covered. A person skilled in the art will be able to recommend what particle concentration to use case by case.

In one embodiment, the lipid has solubility characteristics similar to sebum. In practice, this means that a mixture of compounds having a Hildebrand solubility coefficient between 6.5 and 10 (cal/cm³)^{1/2} are suitable solvents for sebum.

### Additional excipients

In one embodiment, additional excipients are added to the composition.

In one embodiment, the composition further comprises a surfactant. The hydrophilic-lipophilic balance (HLB) of a surfactant is a measure of the degree to which it is hydrophilic or lipophilic. In one embodiment, the HLB of the surfactant is between 9 and 16.

In one embodiment, the surfactant, such as sorbitan laurate, is added in order to prevent sedimentation of the particles, i.e. to maintain a homogenous suspension of the particles.

In one embodiment, the surfactant is selected form the group consisting of Sorbitan laurate, Span 80 and Brij 72. In one embodiment, the surfactant is Sorbitan laurate. The terms sorbitan laurate and span 20 are used herein interchangeably.

In one embodiment, the concentration of Sorbitan laurate is 1 %.

In another embodiment, the surfactant is sugar based. In one embodiment, the sugar based surfactant is selected from the group consisting of sucrose cocoate, sorbitan laurate and polysorbate. In one embodiment, the sugar based surfactant has an HLB value between 9 and 16.

In one embodiment, the particles described above are adjusted to have surface properties making formulation of homogenous suspension possible. The surface properties can be adjusted or optimized by the use of surfactants. In one embodiment, the surfactant is selected from the group of nonionized surfactants that has a HLB, hydrophilic/lipophilic balance of 9 to 16.

In one embodiment, the composition comprises glycerol and/or propylene glycol.

In one embodiment, the composition further comprises a thickener. In one embodiment, glyceryl behenate is added as a thickener to achieve an attractive texture. Other thickeners known in the art may be used. In one embodiment, the thickener is glyceryl behenate or carnauba wax.

In one embodiment, the composition further comprises one or more additional active pharmaceutical ingredients. In one embodiment, the composition further comprises minoxidil. In one embodiment, the composition further comprises finasteride. In one embodiment, the composition further comprises minoxidil and finasteride.

In one embodiment, traditional pharmaceutical compounds that increase viscosity, preservatives and buffers are added to the composition in order to improve physical characteristics of the composition.

### Composition

In one embodiment, the composition is a pharmaceutical composition. In one embodiment, the composition is a cosmetic composition. In on embodiment, the composition is a topical formulation. In one embodiment, the composition is a medicament for topical delivery.

As used herein, the "particle composition" refers to the solid particle present in the formulation while "composition" refers to the composition of the intended product comprising particles and lipid vehicle.

In one embodiment, the composition comprises at least 0.01 wt% peptide, such as at least 0.1 wt% peptide, such as at least 0.5 wt% peptide, such as at least 1 wt% peptide, such as at least 1.5 wt% peptide, such as at least 2 wt% peptide, such as at least 2.5 wt% peptide, such as at least 3 wt% peptide, such as at least 3.5 wt% peptide, such as at least 6 wt% peptide, such as at least 6.5 wt% peptide, such as at least 7 wt% peptide, such as at least 7.5 wt% peptide, such as at least 8 wt% peptide, such as at least 8.5 wt% peptide, such as at least 9 wt% peptide, such as at least 9.5 wt% peptide, such as at least 10 wt% peptide.

In one embodiment, the composition comprises no more than 2 wt% peptide, such as no more than 5 wt% peptide, such as no more than 10 wt% peptide, such as no more than 15 wt% peptide, such as no more than 20 wt% peptide.

In one embodiment, the composition comprises between 0.01 and 5 wt% peptide or peptide derivative, such as between 0.1 and 5 wt% peptide or peptide derivative, such as between 0.1 and 2 wt% peptide or peptide derivative, such as between 0.1 and 1 wt% peptide or peptide derivative, such as between 1 and 5 wt% peptide or peptide derivative, such as between 5 and 10 wt% peptide or peptide derivative, such as between 10 and 15 wt% peptide or peptide derivative, such as between 15 and 20 wt% peptide or peptide derivative. In one embodiment, the composition comprises between 0.01 and 5 wt% peptide, such as between such as between 0.01 and 2 wt% peptide, such as between 0.1 and 1 wt% peptide, such as between 1 and 5 wt% peptide, such as between 5 and 10 wt% peptide, such as between 10 and 15 wt% peptide, such as between 15 and 20 wt% peptide.

In one embodiment, the composition comprises at least 40 wt% lipid, 50 wt% lipid, such as at least 55 wt% lipid, such as at least 60 wt% lipid, such as at least 65 wt% lipid, such as at least 70 wt% lipid, such as at least 75 wt% lipid, such as at least 80 wt% lipid, such as at least 85 wt% lipid, such as at least 90 wt% lipid, such as at least 95 wt% lipid.

In one embodiment, the composition comprises no more than 55 wt% lipid, such as no more than 60 wt% lipid, such as no more than 65 wt% lipid, such as no more than 70 wt% lipid, such as no more than 75 wt% lipid, such as no more than 80 wt% lipid, such as no more than 85 wt% lipid, such as no more than 90 wt% lipid, such as no more than 95 wt% lipid.

In one embodiment, the composition comprises between 40 and 99 wt% lipid, such as between 40 and 60 wt% lipid, such as between 50 and 60 wt% lipid, such as between 60 and 70 wt% lipid, such as between 70 and 80 wt% lipid, such as between 80 and 90 wt% lipid, such as between 90 and 99.95 wt% lipid,

In one embodiment, the composition comprises at least 0.1 wt% saccharide, such as at least 0.5 wt% saccharide, such as at least 1 wt% saccharide, such as at least 1.5 wt% saccharide, such as at least 2 wt% saccharide, such as at least 2.5 wt% saccharide, such as at least 3 wt% saccharide, such as at least 3.5 wt% saccharide, such as at least 6 wt% saccharide, such as at least 6.5 wt % saccharide, such as at least 7 wt% saccharide, such as at least 7.5 wt% saccharide, such as at least 8wt% saccharide, such as at least 8.5 wt% saccharide, such as at least 9wt% saccharide, such as at least 9.5 wt% saccharide, such as at least 10 wt% saccharide.

In one embodiment, the composition comprises no more than 2 wt% saccharide, such as no more than 5 wt% saccharide, such as no more than 10 wt% saccharide, such as no more than 15 wt% saccharide, such as no more than 20 wt% saccharide.

In one embodiment, the composition comprises between 0.01 and 5 wt% saccharide or modified saccharide, such as between 0.01 and 2 wt% saccharide or modified saccharide, such as between 0.1 and 2 wt% saccharide or modified saccharide, such as between 1 and 5 wt% saccharide, such as between 5 and 10 wt% saccharide or modified saccharide, such as between 10 and 15 wt% saccharide or modified saccharide, such as between 15 and 20 wt% saccharide or modified saccharide. In one embodiment, the composition comprises between 0.01 and 0.1 and 1 wt% saccharide, such as between 1 and 5 wt% saccharide, such as between 5 and 10 wt% saccharide, such as between 10 and 15 wt% saccharide, such as between 15 and 20 wt% saccharide.

In one embodiment, the saccharide is sucrose and the lipid is isopropyl myristate. In one embodiment, the composition further comprises petrolatum. In one embodiment, the composition further comprises glyceryl behenate. In one embodiment, the composition further comprises sorbitan laurate. In one embodiment, the composition comprises or consists essentially of the peptide; sucrose; glyceryl behenate; petrolatum; isopropyl myristate; and sorbitan laurate.

It is to be understood that the sum of the components of the total composition does not exceed 100 wt%.

In one embodiment, the composition comprises or consists essentially of about 0.01 to 1 wt% peptide or peptide derivative; 0.01 to 4 wt% saccharide or modified saccharide; 85 to 95 wt% lipid; and optionally 2 to 10 wt% surfactant.

In one embodiment, the composition comprises or consists essentially of about:
a. 0.01 to 1 wt% peptide or peptide derivative;
b. 0.01 to 2 wt% saccharide or modified saccharide;
c. 35 to 50 wt% petrolatum; and
d. 40 to 60 wt% isopropyl myristate.

In one embodiment, the composition comprises or consists essentially of about:
a. 0.01 to 1 wt% peptide or peptide derivative;
b. 0.01 to 2 wt% saccharide or modified saccharide;
c. 1 to 6 wt% glyceryl behenate;
d. 35 to 45 wt% petrolatum;
e. 40 to 60 wt% isopropyl myristate; and
f. 2 to 10 wt% surfactant, such as sorbitan laurate.

In one embodiment, the composition comprises or consists essentially of about:
a. 0.01 to 1 wt% peptide or peptide derivative;
b. 0.01 to 2 wt% sucrose, mannitol or glucose;
c. 1 to 8 wt% glyceryl behenate or carnauba wax;
d. 35 to 45 wt% petrolatum;
e. 40 to 60 wt% isopropyl myristate; and
f. 2 to 10 wt% surfactant, such as sorbitan laurate.

In one embodiment, the composition comprises 40 to 60 wt% isopropylmyristate, 2 to 6 wt% Sorbitan laurate and 2 to 6 wt% glyceryl behenate In one embodiment, the composition comprises 50% isopropylmyristate, 0,2% Sorbitan laurate and 3% glyceryl behenate or 6% carnauba wax. In one embodiment, the lipid comprises isopropyl myristate, petrolatum and glyceryl behenate. In one embodiment, the composition comprises 50 wt% isopropylmyristate, 4 wt% Sorbitan laurate and 3 wt% glyceryl behenate. In one embodiment, the composition comprises 50% isopropylmyristate, 0,2% Sorbitan laurate and 6% carnauba wax.

In one embodiment, the composition comprises or consists essentially of about:
a. 0.01 to 1 wt% peptide or peptide derivative;
b. 0.01 to 2 wt% sucrose;
c. 1 to 6 wt% glyceryl behenate;
d. 35 to 45 wt% petrolatum;
e. 40 to 60 wt% isopropyl myristate; and
f. 2 to 10 wt% sorbitan laurate.

In one embodiment, the composition comprises or consists essentially of about:
a. 0.01 to 1 wt% peptide or peptide derivative;
b. 0.01 to 1 wt% sucrose;
c. 1 to 6 wt% glyceryl behenate;
d. 35 to 45 wt% petrolatum;
e. 40 to 60 wt% isopropyl myristate; and
f. 2 to 6 wt% sorbitan laurate.

In one embodiment, the composition comprises or consists essentially of about:
a. 0.2 wt% peptide;
b. 0.4 wt% sucrose;
c. 3 wt% glyceryl behenate;
d. 42.4 wt% petrolatum;
e. 50 wt% isopropyl myristate; and
f. 4 wt% sorbitan laurate.

In one embodiment, the sum of the amounts in percent of the components does not exceed 100 %.

In one embodiment, the composition comprises or consists essentially of about:
a) 1 wt% peptide;
b) 2 wt% sucrose;
c) 95 wt% lipid; and optionally
d) 2 wt% Sorbitan laurate .

In one embodiment, the composition is essentially a water free composition.

In one embodiment, the composition is in the form of an ointment, a powder, a spray, a lotion, a gel, foam, a cream, make-up or a shampoo. In one embodiment, the composition is in the form of a powder.

### Method of manufacturing

The particles can be manufactured by standard techniques such as freeze drying, spray drying or freeze spraying, followed by operations to reduce particle size to submicron levels. Such reduction of size can be performed using standard grinding techniques such as ball milling. Other suitable techniques are emulsification and solvent evaporation and yet other techniques for generation of particles can use precipitation of the active agent/saccharide.

In one aspect, the present invention provides a method of manufacturing a composition as described herein, comprising the following steps:
a) mixing the peptide with a saccharide;
b) freeze drying the mixture of a);
c) mixing b) with a lipid and a surfactant;
d) grinding the mixture of c); and
e) optionally mixing the mixture of d) with a lipid and a thickener.

Freeze-drying of the peptide together with sucrose may be performed to increase the dissolution of the peptide particles and to increase the chemical stability of the peptide.

In one embodiment, the method of manufacturing the composition as described herein comprises the following steps:
a) mixing the peptide together with the saccharide, such as sucrose;
b) lyophilizing the mixture of a);
c) mixing the lipid, such as isopropyl myristate, with a surfactant, for example sorbitan laurate, to obtain a homogenous solution;
d) adding the peptide-saccharide mixture of b) to the lipid-surfactant mixture in c) to obtain a suspension;
e) grinding the mixture of d), for example by using a standardized bead, wet-milling method;
f) mixing a lipid, such as petrolatum, with a surfactant, such as sorbitan laurate, and a thickener, such as glyceryl behenate; and
g) mixing the mixture of e) with the mixture of f).

### Area of delivery

The present invention relates to a composition suitable for topical delivery of one or more active agents, such as a peptide. In one embodiment, the topical application site is on a skin surface of the patient. In one embodiment, the topical application site is on a tissue surface of a patient. In one embodiment, the invented product is intended for delivery of medically active substances into follicles or into skin surrounding the follicle.

In one embodiment, the composition as described herein is for stimulating hair growth in a mammal and is not limited to use on the scalp, but may also be applied elsewhere on the body (including the face to encourage the growth of a beard, eyelashes, eyebrows, *etc*.)

### Use of the composition

In one aspect, the present invention provides a method for topical delivery of the peptide or variant/fragment to a subject in need thereof, comprising applying an effective amount of the said composition to a topical application site of the subject.

In one embodiment, the composition may be applied for several days, for example for several weeks, such as several months.

The present invention is not limited to medical uses but the composition as described herein may also be used as cosmetic agents (in the sense that it does not provide any physical health improvement, as such, but merely provide an aesthetic benefit to the mammal).

It will be further appreciated by skilled persons that the compositions of the invention may be used *in vivo, ex vivo* or *in vitro.* For example, when using the composition for stimulating hair growth, the composition may be used to stimulate hair growth ex *vivo,* for example in a skin explant prior to grafting of the skin on to the mammal.

In one embodiment, the composition is applied to a subject or a patient. In one embodiment, the subject or patient is a mammal. In one embodiment, the mammal is selected from the group consisting of a human, a dog, a cat and a horse. In one embodiment, the mammal is a human.

The composition may have a wound healing anti-aging, anti-irritating, anti-pruritic, antibacterial, antifungal, antiviral, anti-inflammatory, anti-allergic, anti-wrinkle and/or anti-acne effect.

### Medical use

In one aspect, the present invention is related to the composition as described herein for use as a medicament. In one embodiment, the peptide of the pharmaceutical composition is the active ingredient, such as the active pharmaceutical ingredient. Thus, in one embodiment, the pharmaceutical composition is suitable for use in the treatment of the indications, which the peptide is effective against.

In one embodiment, the composition as described herein is for use in the treatment of dermatological conditions. In one aspect, the present invention provides a method for treating dermatological conditions. In one embodiment, the composition is for use in the treatment of dermatological conditions. In one embodiment, the dermatological condition is selected from the group consisting of psoriasis, atopic dermatitis, eczema, precarcinogenic states and skin infections. Precarcinogenic states encompass skin lesions that are pre-cancerous. Skin infections may be bacterial, viral, fungal and parasitic infections.

In aspect, the present invention relates to use of the composition as described herein in the manufacture of a medicament for treatment or prevention of a disease or condition associated with hair loss.

In aspect, the present invention relates to use of the composition as described herein in the manufacture of a medicament for treatment of dermatological conditions.

In one embodiment, an effective amount of the composition is administered do the subject. As used herein, the term 'effective' means adequate to accomplish a desired, expected, or intended result. For instance, an 'effective amount' or a 'cosmetically effective amount' or a 'therapeutically effective amount' means an amount that is adequate to accomplish a desired, expected or intended result. This is a predetermined quantity of active material calculated to produce the desired therapeutic effect. As is appreciated by those skilled in the art, the amount of a compound may vary depending on its specific activity. Suitable dosage amounts may contain a predetermined quantity of active composition calculated to produce the desired therapeutic effect in association with the required diluent. In the methods and use for manufacture of compositions of the invention, a therapeutically effective amount of the active component is provided. A therapeutically effective amount can be determined by the ordinary skilled medical or veterinary worker based on patient characteristics, such as age, weight, sex, condition, complications, other diseases, *etc*., as is well known in the art.

As used herein, a `therapeutically effective amount', or 'effective amount', or 'therapeutically effective', refers to that amount of active ingredient, which ameliorates the symptoms or condition. For example, in one embodiment, a `therapeutically effective amount' refers to that amount which provides a stimulatory effect on hair growth.

### Hair loss

In one embodiment, the composition as described herein is for use in the treatment or prevention of a disease or condition associated with hair loss.

In one aspect, the present invention provides a method for stimulating hair growth, said method comprising administering topically to a patient in need thereof, an effective amount of the said composition.

In one aspect, the present invention provides a method for treating hair loss, said method comprising administering topically to a patient in need thereof, an effective amount of the said composition.

In one embodiment, the composition as described herein is for use in the treatment of alopecia. Alopecia is typically associated with the loss of anagen hairs. However, it will be appreciated that the compositions of the invention may also be used for treatment of conditions associated with the loss of telogen hairs.

In one embodiment, the alopecia is selected from the group consisting of:
(a) androgenic alopecia (also known as androgenetic alopecia, *alopecia androgenetica*, male pattern baldness or female pattern baldness);
(b) traction alopecia;
(c) anagen effluvium;
(d) telogen effluvium;
(e) alopecia areata;
(f) alopecia totalis;
(g) alopecia universalis;
(h) alopecia barbae;
(i) alopecia mucinosa;
(j) alopecia neoplastica;
(k) cicatricial alopecia; and
(l) scarring alopecia.

For example, the alopecia may be androgenic alopecia.

Alternatively, the alopecia may be anagen effluvium. This condition, resulting from the early entry of hairs into the telogen phase, may be due to a variety of causes, including eating disorders, fever, childbirth, chronic illness, major surgery, anemia, severe emotional disorders, crash diets, hypothyroidism, and drugs.

Thus, in one embodiment, the hair loss is induced by radiotherapy and/or chemotherapy agents. For example, hair loss is a common and distressing side effect of treatment with chemotherapeutic drugs such as cisplatin, etoposide and paclitaxel.

In one embodiment, the modified osteopontin peptides present in the compositions of the invention are capable of stimulating hair growth in mammals.

In one embodiment, the peptide is capable of stimulating the growth of human hair.

In a further embodiment, the peptide is capable of stimulating the growth of hair *in vivo.*

It will be appreciated by persons skilled in the art that the stimulation of hair growth may be mediated by an effect of existing hair follicles and/or by inducing the formation of new hair follicles. Thus, in one embodiment, the modified osteopontin peptide is capable of stimulating existing hair follicles (for example, by prolonging the anagen phase and/or by shortening the telogen phase such that the resting follicles become active).

In a further embodiment, the peptide is capable of inducing the formation of new hair follicles, or stem cells for producing the same.

### Cosmetic use

In one aspect, the composition as described herein is for use, wherein the use is cosmetic.

In one aspect, the present invention provides the use of a composition as described herein for stimulating hair growth in a mammal, wherein the use is cosmetic. In one embodiment, the cosmetic composition is for stimulating existing hair follicles and/or inducing the growth of new hair follicles (or stem cells for producing the same).

In one embodiment, the cosmetic composition is used for the treatment or prevention of baldness, which may be associated with a receding hairline and/or thinning hair.

### Combination treatment

It will be appreciated by persons skilled in the art that the compositions of the invention may be used on their own or in combination with other therapeutic or cosmetic agents. For example, the compositions of the invention may be used in a combination therapy with existing treatments to prevent loss of existing hair and/or to stimulate growth of new hair, for example potassium channel openers, such as minoxidil (Regaine RTM., Pharmacia Corp.) and diazoxide; 5-alpha-reductase inhibitors, such as finasteride (Propecia RTM., Merck & Co.); and the immunosuppressant cyclosporin A.

### Examples

### Example 1. FOL-005 solubility and stability testing.

The most promising topical delivery route for FOL-005 is the sebum route. The objective of this study was to determine the solubility and compatibility of FOL-005 in potential excipients and artificial sebum.

### Materials and Methods

**Table 1. Chemicals used for solubility and compatibility testing.**

| | |
|---|---|
| Vehicle excipients | Paraffin oil |
| | Glycerol |
| | Propylene glycol |
| | Lactic acid |
| Sugars | Dextran |
| | Mannitol |
| | Sucrose |
| | Glucose |
| Surfactants | Cithrol |
| API | FOL-005 Na-salt |
| | FOL-005 Ac-salt |
| | Beta-Asp FOL-005 (degradation product) |

**Table 2. Chemicals used for preparing artificial sebum.**

| |
|---|
| Olive oil |
| Coconut oil |
| Cottonseed oil |
| Squalene |
| cholesterol |
| Vitamin E |

### Results

### Solubility of FOL-005 in vehicle excipients and artificial sebum

The solubility of FOL-005 was tested in vehicle excipients at room temperature and in artificial sebum at 37°C. The testing was performed by charging 0.1% (w/w) of FOL-005 together with 99.90% excipient. If FOL-005 did not dissolve the double amount of excipient was added. The mixtures were stirred with magnetic stirrer for 2 hours. If particles of FOL-005 remained, the mixture was centrifuged at14000 rpm or 10000 rpm (artificial sebum) for 5 minutes and the supernatant was withdrawn for analysis.

The concentration of FOL-005 was determined by HPLC analysis in all batches.

**Table 3a. Composition of vehicle batches with FOL-005 manufactured for solubility testing.**

| **Batch no** | ISM16 085 A | ISM16 085 B | ISM16 085 C | ISM16 085 D | ISM16 087 A | ISM16 087 B | ISM16 087 C | ISM16 087 D | ISM16 087 E |
|---|---|---|---|---|---|---|---|---|---|
| **Ingredi ent** | **(% w/w)** | | | | | | | | |
| FOL-005 (85.9%) | 0.05 | 0.05 | 0.05 | 0.10 | 0.11 | 0.11 | 0.11 | 0.11 | 0.10 |
| Paraffin oil | 99.95 | | | | | | | | |
| Glycero l | | 99.95 | | | | | | | |
| Propyle ne glycol | | | 99.95 | | | | | | |
| Lactic acid | | | | 99.90 | | | | | |
| 10% Glucos e in water | | | | | 99.89 | | | | |
| 30% Glucos e in water | | | | | | 99.89 | | | |
| 10% Mannito l in water | | | | | | | 99.89 | | |
| 10% Sucros e in water | | | | | | | | 99.89 | |
| 30% Sucros e in water | | | | | | | | | 99.90 |
| Total | 100.0 | 100.0 | 100.0 | 100.0 | 100.0 | 100.0 | 100.0 | 100.0 | 100.0 |
| Observ ation | not compl etely soluble | not compl etely soluble | not compl etely soluble | solubl e | solubl e | solubl e | solubl e | solubl e | solubl e |
| **FOL-005 HPLC assay (mg/ml)** | 0 | 0.64 | 0.49 | 1.17 | 1.01 | 1.07 | 1.04 | 1.04 | 1.04 |
| **FOL-005 solubili ty (mg/ml)** | 0 | 0.64 | 0.49 | >1.17 | >1.01 | >1.07 | >1.04 | >1.04 | >1.04 |

**Table 3b. Results from solubility test of FOL-005 (sodium salt) in vehicle excipients and artificial sebum.**

| **Batch no** | ISM16085A | ISM16091 | ISM16100 |
|---|---|---|---|
| **Ingredient** | **(% w/w)** | | |
| FOL-005 (85.9%) | **0.11** | **0.05** | **0.05** |
| PBS buffer pH 7.4 | 99.89 | | |
| Artificial sebum ISM16089 (37°) | | 99.95 | |
| 0.5% Cithrol in paraffin oil | | | **99.95** |
| **Total** | **100.0** | **100.0** | **100.0** |
| **Observation** | soluble | not completely soluble | not completely soluble |
| **FOL-005 HPLC assay mg/ml** | **0.98** | **0.27** | **0.01** |
| **FOL-005 solubility mg/ml** | **>0.98** | **0.27** | **0.01** |

### Conclusion

Solubility of FOL-005 (sodium salt) at room temperature:
- solubility in paraffin oil with cithrol 0.01mg/ml
- solubility in glycerol 0.31mg/ml
- solubility in propylene glycol 0.16mg/ml
- solubility in sugar solutions of glucose, mannitol and sucrose > 1mg/ml
- solubility in PBS buffer >1mg/ml
- solubility in artificial sebum at 37°C 0.27mg/ml

FOL-005 (sodium salt) in sugar solutions has good chemical stability in water solutions with glucose and sucrose when stored at 2-8°C for 7 weeks. No change in the content of FOL-005 could be seen in water solution with 10% glucose or 10% sucrose when stored at 2-8°C for 7 weeks, while the content FOL-005 in 10% glucose solution decreased from 0.9 mg/ml to 0.7 mg/ml and the content of FOL-005 in 10% sucrose solution decreased 0.9 mg/ml to 0.04 mg/ml when stored at room temperature.

The chemical stability of FOL-005 was found to be poorer in water solutions with mannitol when stored at 2-8°C for 7 weeks compared to sucrose and glucose. The content of FOL-005 in 10% mannitol solution decreased from 0.9 mg/ml to 0.8 mg/ml when stored at 2-8°C for 7 weeks and from 0.8 mg/ml to 0.2 mg/ml when stored at room temperature for 7 weeks.

FOL-005 has good chemical stability in glycerol. No change in the content of FOL-005 could be seen when stored at 2-8°C for 7 weeks, while the FOL-005 content decreased from 0.3 mg/ml to 0.2 mg/ml when stored at room temperature for 7 weeks.

The chemical stability of FOL-005 was found to be poorer in propylene glycol. The content FOL-005 decreased from 0.16 mg/ml to 0.15 mg/ml when stored at 2-8°C for 7 weeks and from 1.6 mg/ml to 0.07 mg/ml when stored at room temperature for 7 weeks.

The solubility of FOL-005 in 10% glucose solution was determined to be above 4.38 % (w/w).

### Example 2. FOL-005 stability testing.

### Materials and Methods

### Preparation of FOL-005 suspension studied in a screening stability study

Manufacturing method - suspension:
- Charge Aerosil into a beaker
- Add paraffin oil to the beaker and mix carefully
- Stir until a homogenous viscous gel is formed
- Heat to 70°C under stirring
- Homogenize, at 70°C, until a homogenous gel is obtained
- Cool to room temperature
- Add isopropyl myristate
- Stir until a homogenous suspension is formed
- Charge FOL-005 sucrose particles and Sorbitan laurate into a new beaker
- Add the suspension into the beaker with FOL-005 sucrose particles and Sorbitan laurate
- Homogenize, at 70°C, until a homogenous suspension is formed

### Results

**Table 4a. Results from stability testing of FOL-005 (sodium salt) in three suspensions.**

| | **Batch no** | **ISM16192** | **ISM16193** | **ISM16194** |
|---|---|---|---|---|
| | | **S1** | **S2** | **S3** |
| | **Ingredient** | % (w/w) | | |
| | FOL-005 sucrose 1:1 pH 7 | 0.20 | 0.20 | 0.20 |
| | (FOL-005)* | (0.086) | (0.086) | (0.086) |
| | Sorbitan | - | - | 0.02 |
| | laurate | 96.80 | 86.74 | 86.74 |
| | Paraffin oil | - | 10.04 | 10.04 |
| | Isopropyl myristate | 3.00 | 3.01 | 3.00 |
| | Aerosil R972 | **100.0** | **100.0** | 100.0 |
| | Total | | | |
| **Initial analysis** | **FOL-005 HPLC assay recovery %** | 57.3¹ | 77.5¹ | 106.7¹ |
| | **Sum related subst., % rel. area** | Not detected² | Not detected² | Not detected² |
| | **beta-asp, RRT= 0.96** | Not detected² | Not detected² | Not detected² |
| **4 weeks, 25°C** | **FOL-005 HPLC assay recovery %** | 85.9⁴ | 73.2 | 106.2 |
| | **Sum related subst., % rel. area** | 7.22 | 4.93 | 3.55 |
| | **beta-asp, RRT= 0.96** | Not detected | Not detected | Not detected |
| | **RRT= 0.98** | 3.42 | 0.15 | Not detected |
| **10 weeks, 25°C** | **FOL-005 HPLC assay recovery %** | 92.5 | 98.7 | 110.2 |
| | **Sum related subst., % rel. area** | 10.13 | 6.11 | 4.04 |
| | **beta-asp, RRT= 0.96** | Not detected | Not detected | Not detected |
| | **RRT= 0.98** | 5.30 | 0.34 | 0.12 |
| | **RRT=1.09** | 1.64 | 1.55 | 2.31 |
| | **RRT= 1.21** | 1.47 | 2.48 | Not detected |
| **4 weeks, 30°C** | **FOL-005 HPLC assay recovery %** | 90.1 | 97.5 | 80.8 |
| | **Sum related subst., % rel. area** | 9.81 | 4.74 | 4.15 |
| | **beta-asp, RRT= 0.96** | Not detected | Not detected | Not detected |
| | **RRT= 0.98** | 5.83 | 0.17 | 0.13 |

| | | | | |
|---|---|---|---|---|
| * FOL-005 sucrose 1:1 contains 45.8 % FOL-005 ¹Different sample preparation steps in the analytical method were evaluated. The data given in the Table is from the analysis giving the highest recovery. ²Rel. substances could not be detected due to too small sample quantity (0.25 g sample per 25 ml solution). ³Sample preparation tetrahydrofuran and water ⁴Sample preparation Span, water and acetonitrile | | | | |

### Conclusion

Suspensions:
- The recovery of FOL-005 in S3 was low when stored at 25°C. It was however increased when the analytical sampling preparation was improved.
- No beta-ASP (degradation product of FOL-005) was detected in the three suspensions after 4 and 10 weeks storage at 25°C and 4 weeks storage at 30°C.
- Low amounts of degradation product RRT=0.98 was seen in S2 and S3, while higher amounts were seen in S1.
- It was concluded that the chemical stability of FOL-005 looks promising in S2 and S3.

Tables 4b-d show the purity of FOL-005. Data is also shown in Figures 1-3.

**Table 4b. Purity of FOL-005 in different pH.**

| **Purity FOL-005 (%)** | | | | | | |
|---|---|---|---|---|---|---|
| weeks | months | buffer pH 5 | buffer pH 6 | buffer pH 7 | buffer pH 7.6 | water free |
| 0 | 0 | 98,66 | 98,64 | 98,85 | 99 | 98,17 |
| 1 | 0,25 | 92,63 | 96,06 | 97,42 | 97,15 | |
| 4 | 1 | 78 | 90 | 92 | 92 | 99,06 |
| 8 | 2 | 65,42 | 84,53 | 87,84 | 87,83 | 98,04 |
| 12 | 3 | 55,44 | 76,89 | 82,96 | 82,65 | 98,33 |
| | 6 | | | | | 98,34 |
| | 9 | | | | | 98,22 |
| | 12 | | | | | 97,7 |

**Table 4c. Purity of FOL-005 with different conditions of isopropyl myristate (IPM).**

| **Purity FOL-005 (%)** | | | | | | | |
|---|---|---|---|---|---|---|---|
| Months | 0 | 1 | 2 | 3 | 6 | 9 | 12 |
| Un-Dried IPM 2-8C | 99,04 | 99,28 | | 98,84 | 99,04 | | 99 |
| Dried IPM 2-8C | 98,17 | 98,18 | | 98,13 | 97,95 | | 97,81 |
| Un-Dried IPM 25C | 99,04 | 98,48 | 98,16 | 97,04 | 96,73 | 95,77 | 95,57 |
| Dried IPM 25C | 98,17 | 99,06 | 98,04 | 98,33 | 98,34 | 98,22 | 97,7 |
| Un-Dried IPM 30C | 99,04 | 98,01 | 97,68 | 96,24 | 95,91 | 94,24 | 94,59 |
| Dried IPM 30C | 98,17 | 97,38 | | | 97,74 | | 96,86 |

**Table 4d. Purity of FOL-005 with or without sucrose at different temperatures.**

| **Purity FOL-005 (%)** | | | | | | | | |
|---|---|---|---|---|---|---|---|---|
| Months | | 0 | 1 | 2 | 3 | 6 | 9 | 12 |
| Sucrose 2-8C | | 99,04 | 99,28 | | 98,84 | 99,04 | | 99 |
| Sucrose 25C | | 99,04 | 98,48 | 98,16 | 97,04 | 96,73 | 95,77 | 95,57 |
| Sucrose 30C | | 99,04 | 98,01 | 97,68 | 96,24 | 95,91 | 94,24 | 94,59 |
| | | | | | | | | |
| Na | 2-8C | 98,99 | 99,13 | | 98,23 | 98,8 | | 98,96 |
| Na | 25C | 98,99 | 98,29 | 97,17 | 95,17 | 93,1 | 92,86 | 92,7 |
| Na | 30C | 98,99 | 96,82 | 95,74 | 94,06 | 93,58 | 91,79 | 90,47 |

### Conclusion

It is demonstrated that FOL-005 was degraded at various pH, whereas the degradation was very limited in water free conditions. Further, dried IPM resulted in less degradation than un-dried IPM of FOL-005 at 25 and 30 °C, and sucrose particles resulted in less degradation than Na salt of FOL-005 at 25 and 30 °C.

### Example 3. Lyophilization of FOL-005 with mannitol

The possibility to make particles of FOL-005 (acetate-salt) by freeze drying and test the compatibility between peptide and a prototype vehicle.

### Materials and Methods

The following chemicals were used in this study:
Mannitol, Sodium hydroxide, HCl (37%), API, FOL-005 Ac-salt, Beta-Asp FOL-005 (degradation product), Deionized water, Paraffin oil, Glycerol, Propylene glycol, Cithrol^{™}DPHS, Tween 60 BergaBest MCT oil 60/40.

### Preparation of solution for freeze drying test

The mixtures given in Table 5a were prepared for freeze drying. The mixtures were dispensed into 2 ml glass vials. The number of glass vials per mixture is given in the table below. Each vial contains approximately 2 mg FOL-005.

**Table 5a. Composition mixtures prepared for freeze drying**

| | FOL-005: | FOL-005: |
|---|---|---|
| | Mannitol 2:1, pH 7.1 | Mannitol 1:1, pH 7.1 |
| Ingredient | % | % |
| FOL-005 Ac-salt | 0.10 | 0.11 |
| Mannitol | 0.05 | 0.10 |
| Sodium hydroxide 0.1 M to pH 7,4 | 1.62 | - |
| Sodium hydroxide 1 M to pH 7,4 | - | 0.20 |
| HCl 0.1 M to pH 3.5 | - | 0.14 |
| Water | 98.23 | 99.46 |
| Total | 100.00 | 100.00 |
| pH | 7.5 | 7.5 |
| Number of vials | 22 | 22 |
| | | |

| Batch no | ISM16111: | ISM16113: |
|---|---|---|
| | FOL-005: Mannitol 2:1 pH 3.5 | FOL-005: Mannitol 1:1, pH 3.5 |
| Ingredient | | |
| ISM16111 | 98 | |
| ISM16113 | | 98 |
| HCl 0.1 M to pH 3.5 | 2 | 2 |
| Total | 100 | 100 |
| pH | 3.5 | 3.5 |
| Number of vials | 3 | 3 |

| | | |
|---|---|---|
| *pH tends to decrease with time. pH was determined to 7.1 before freeze drying. | | |

### Freeze drying conditions

Vials were first stored at -35°C for 2 hours and then transferred quickly to the Freeze dryer. Vials were freeze dried under vacuum at -55°C for 24 hours.

### Preparation of formulations for compatibility testing

The compatibility between FOL-005 powder and vehicle excipients was tested in pure paraffin oil and in one potential formulation. The composition of the formulations is presented in Table 5b.

**Table 5b. Formulations for compatibility testing of freeze dried powder.**

| Ingredient | Formulation A % (w/w) | Formulation B % (w/w) |
|---|---|---|
| Paraffin oil | 100.00 | 15.50 |
| Glycerol | - | 44.83 |
| Propylene glycol | - | 30.10 |
| Cithrol^{™}DPHS | - | 2.64 |
| Tween 60 | - | 2.61 |
| BergaBest MCT oil | | |
| 60/40 | - | 4.33 |
| Total | 100.00 | 100.00 |

### Assay method

The test items were analyzed using the HPLC-UV method Assay of FOL-005 in formulation by HPLC. The concentration of FLO-005 was determined and beta-asp FOL-005 as well.

### Results

The results from the HPLC analysis of freeze dried powder of FOL-005 in shown in Table 6a-c.

**Table 6a. Results from HPLC analysis of freeze dried powders of FOL-005 (acetate salt) and mannitol, initial analysis and after 4 weeks storage at 2-8°C and room temperature.**

| | Batch | ISM16113 FOL-005: Mannitol 1:1, pH 3.5 | ISM16113 FOL-005: Mannitol 1:1, pH 7.1 | ISM16111 FOL-005: Mannitol 2:1, pH 3.5 | ISM16111 FOL-005: Mannitol 2:1, pH 7.1 |
|---|---|---|---|---|---|
| Initial analysis | FOL-005 in weight % | 43.6 | 44.6 | 52.8 | 59.1 |
| | Sum related subst., % rel. area | 5.61 | 4.91 | 5.02 | 4.81 |
| | beta-asp, RRT= 0.96 | 0.34 | 0.29 | 0.32 | 0.32 |
| 4 weeks, 2-8°C | FOL-005 in weight % | 44.4 | 45.9 | 59.4 | 61.3 |
| | Sum related subst., % rel. area | 3.53 | 3.49 | 3.27 | 3.73 |
| | beta-asp, RRT= 0.96 | 0.46 | 0.50 | 0.51 | 0.51 |
| | RRT= 1.04 | 0.37 | 0.32 | 0.39 | 0.33 |
| | RRT=1.05 | na | na | na | na |
| 4 weeks, room temperature | FOL-005 in weight % | - | 44.7 | - | 59.5 |
| | Sum related subst., % rel. area | - | 6.30 | - | 6.20 |
| | beta-asp, RRT= 0.96 | - | 031 | - | 0.50 |
| | RRT= 1.04 | - | 0.46 | - | 0.45 |
| | RRT=1.05 | - | 2.09 | - | 2.13 |

**Table 6b. Results from HPLC analysis of freeze dried powder of FOL-005 (acetate salt) and mannitol in paraffin oil (formulation A), initial analysis and after 4 weeks storage at 2-8°C and room temperature.**

| | Batch | FOL-005: Mannitol 1:1, pH 3.5 + | FOL-005: Mannitol 1:1, pH7.1 + | FOL-005: Mannitol 2:1, pH3.5 + | FOL-005: Mannitol 2:1, pH7.2 + |
|---|---|---|---|---|---|
| | | Formulation A | Formulation A | Formulation A | Formulation A |
| Initial analysis | FOL-005 in weight % | 0.077 | 0.092 | 0.085 | 0.087 |
| | Sum related subst., % rel. area | 4.23 | 4.51 | 4.85 | 4.27 |
| | beta-asp, RRT= 0.96 | 0.22 | 0.21 | 0.22 | 0.23 |
| 4 weeks, 2-8°C | FOL-005 in weight % | 0.097 | 0.096 | 0.088 | 0.090 |
| | Sum related subst., % rel. area | 3.36 | 2.99 | 2.81 | 2.78 |
| | beta-asp, RRT= 0.96 | 0.60 | 0.26 | 0.26 | 0.24 |
| | RRT= 1.04 | 0.55 | 0.35 | 0.41 | 0.46 |
| 4 weeks, room temperature | FOL-005 in weight % | - | 0.092 | - | 0.091 |
| | Sum related subst., % rel. area | - | 2.90 | - | 3.16 |
| | beta-asp, RRT= 0.96 | - | 0.40 | - | 0.32 |
| | RRT= 1.04 | - | 0.42 | - | 0.45 |

**Table 6c. Results from compatibility testing of freeze dried powder of FOL-005 (acetate salt) and mannitol in formulation B, initial analysis and after 4 weeks storage at 2-8°C and room temperature.**

| | Batch | FOL-005: Mannitol 1:1, pH 3.5 + Formulation B | FOL-005: Mannitol 1:1, pH 7.1 + Formulation B | FOL-005: Mannitol 2:1, pH 3.5 + Formulation B | FOL-005: Mannitol 2:1, pH 7.2 + Formulation B |
|---|---|---|---|---|---|
| Initial analysis | FOL-005 in weight % | na | na | 0.085 | 0.073 |
| | Sum related subst., % rel. area | na | na | 5.96 | 7.52 |
| | beta-asp, RRT= 0.96 | na | na | 0.25 | 0.28 |
| 4 weeks, 2-8°C | FOL-005 in weigth % | 0.057 | 0.067 | 0.080 | 0.068 |
| | Sum related subst., % rel. area | 7.36 | 9.92 | 6.08 | 9.68 |
| | beta-asp, RRT= 0.96 | na | na | na | na |
| | RRT= 1.04 | 5.27 | 7.34 | 3.08 | 7.32 |
| 4 weeks, room temperature | FOL-005 in weight % | - | 0.024 | - | 0.027 |
| | Sum related subst., % rel. area | - | 50.99 | - | 50.76 |
| | beta-asp, RRT= 0.96 | - | na | - | na |
| | RRT= 1.04 | - | 36.59 | - | 36.97 |

### Conclusion

The acetate salt of FOL-005 was successfully freeze dried with mannitol as stabilizer. Two different pH of the lyophilisation mixtures, 3.5 and 7.1, and two different ration between FOL-005 and mannitol, 1:1 and 2:1, were tested. No difference in the chemical stability of the freeze dried powders due to pH or ration between FOL-005 and mannitol could be seen when stored at 2-8°C for 4 weeks.

The freeze dried powder of FOL-005 and mannitol was chemically stable when stored at 2-8°C for 4 weeks. When stored at room temperature for 4 weeks an increase in the sum of related substances could be seen from 5 to 6%.

The freeze dried powder of FOL-005 was found to be chemically stable when dispersed in formulation A, containing 100% paraffin oil, and stored at 2-8 °C and room temperature for 4 weeks.

The chemical stability was found to be poorer in formulation B, containing paraffin oil (16%), glycerol (45%), propylene glycol (30%), Cithrol (3%), Tween 60 (3%) and BergaBest MCT oil (4%). When stored at 2-8°C for 4 weeks no change in the content of FOL-005 could be seen, but an increase in the amount of related substances could be seen, from 7.5 to 10%, as well as a difference in the pattern of related substances. When stored at room temperature for 4 weeks the content of FOL-005 decreased 0.07% to 0.03% and the sum of related substances increased from 7.5 to 50.8.

### Example 4: Thickening of placebo formulations.

In this study, we have investigated the positivity to form viscous formulations with petrolatum and high concentration of isopropylmyristate.

### Materials and methods

### Petrolatum/isopropylmyristate formulations

Petrolatum/isopropylmyristate mixtures were visually inspected to determine if formulations with high viscosity could be made using these two components. The compositions of the formulations manufactured are given in Table 7.

**Table 7. Formulation compositions % (w/w).**

| Ingredients | ISM17165 | ISM17166 | ISM17167 | ISM17168 |
|---|---|---|---|---|
| Isopropylmyristate | 50 | 30 | 20 | 10 |
| Petrolatum | 50 | 70 | 80 | 90 |
| Total | 100 | 100 | 100 | 100 |

### Addition of thickening agents

In order to increase the viscosity of Petrolatum/isopropylmyristate formulations, thickening agents were added and formulations were visually inspected. Formulations compositions are given in Table 8.

**Table 8. Formulation compositions % (w/w).**

| Ingredients | ISM17169 | ISM17170 | ISM 17171 | ISM17172 |
|---|---|---|---|---|
| Isopropylmyristate | 50 | 30 | 50 | 30 |
| Petrolatum | 43.8 | 63.8 | 46.8 | 66.8 |
| Carnauba wax 2442 | 6 | 6 | - | |
| Glyceryl behenate | - | - | 3 | 3 |
| Sorbitan laurate | 0.2 | 0.2 | 0.2 | 0.2 |
| Total | 100 | 100 | 100 | 100 |

### Placebo formulations with sucrose particles

Centrifugation of formulations containing sucrose particles was performed in order to investigate particle sedimentation. 2 % (w/w) sucrose were added to ISM17167-ISM17172 in Table 7 and 8 and 1 % (w/w) sucrose were added to ISM17166. The formulations were mixed before they were centrifuged at 1000 rpm for 3 min and visually inspected.

### Formulations with FOL-005 particles

Particle sedimentation in selected formulations were also investigated by analysing the content of FOL-005 at different positions in tubes with centrifuged formulations. 0.2 % (w/w) FOL-005 were added to the placebo formulations according to Table 9, the formulations were then mixed by magnetic stirring for 2 hours. Each formulation was distributed into two Eppendorf tubes whereof one was centrifuged for 3 min at 1000 rpm. Tubes were stored in a refrigerator until the content of FOL-005 was assayed. The analysis was performed by high-pressure liquid chromatography (RP-HPLC) and ultra violet detection (UV). The compounds were monitored at 220 nm. The unidentified related substances were quantified as % relative area. FOL-005 salt (sodium) was used as external standard.

**Table 9. Formulation compositions % (w/w)**

| Ingredients | ISM17180 | ISM17181 |
|---|---|---|
| FOL-005 (Na-salt) | 0.2 | 0.2 |
| Isopropylmyristate | 49.9 | 49.9 |
| Petrolatum | 43.7 | 46.7 |
| Carnauba wax 2442 | 6 | - |
| Glyceryl behenate | - | 3 |
| Sorbitan laurate | 0.2 | 0.2 |
| Total | 100 | 100 |

### Results

### Petrolatum/isopropylmyristate formulations

As described in Table 10 it was not possible to create nice viscous formulations with only isopropylmyristate and petrolatum. Formulations with 50 % (w/w) or less petrolatum had a low viscosity while formulations with 70 % (w/w) or higher content of petrolatum showed a tendency for phase separation.

**Table 10. Formulation properties.**

| | ISM17165 | ISM17166 | ISM17167 | ISM17168 |
|---|---|---|---|---|
| Isopropylmyristate | 50 | 30 | 20 | 10 |
| Petrolatum | 50 | 70 | 80 | 90 |
| Viscosity | low | medium | medium | high |
| Appearance | OK | Tendency for separation | Tendency for separation | Tendency for separation |

### Addition of thickening agents

Thickening agents were added to the formulations in order to increase the viscosity. In addition, 0.2% (w/w) Sorbitan laurate were added to the formulations as it has a stabilizing effect on FOL-005 particles. As seen in Table 11 addition of Carnauba wax and Glyceryl behenate had positive effect on the formulations. By adding these ingredients formulations with medium to high viscosity could be created.

**Table 11. Formulation properties.**

| | ISM17169 | ISM17170 | ISM17171 | ISM17172 |
|---|---|---|---|---|
| Isopropylmyristate | 50 | 30 | 50 | 30 |
| Petrolatum | 43.8 | 63.8 | 46.8 | 66.8 |
| Carnauba wax 2442 | 6 | 6 | - | |
| Glyceryl behenate | - | - | 3 | 3 |
| Sorbitan laurate | 0.2 | 0.2 | 0.2 | 0.2 |
| Viscosity | medium | high | medium | medium |
| Appearance | OK | OK | OK | Tendency for separation |

### Conclusion

Mixtures of only isopropylmyristate and petrolatum were found to have either a low viscosity or showed a tendency for phase separation. However, by adding Glyceryl behenate or carnauba wax, the formulations showed good appearance and medium viscosity.

The particle sedimentation in the formulations with carnauba wax or glyceryl behenate was investigated and FOL-005 did not appear to sediment in these formulations. Therefore, petrolatum formulations containing 50% (w/w) isoprpylmyristate 0.2% (w/w) Sorbitan laurate and 3% (w/w) glyceryl behenate or 6% (w/w) carnauba wax appeared to be promising alternative for FOL-005 particle suspensions.

### Example 5. Ex vivo testing of formulations of FOL-005 particles

### Materials and Methods

The compositions which were tested ex *vivo* are given in Table 12.

**Table 12a. Compositions tested ex vivo in experiment 1.**

| **Batch** | ISM17209 "FOL-005/sucrose" | ISM17216 "FOL-005/Na" | ISM17213 "Placebo" |
|---|---|---|---|
| **Ingredient, % (w/w)** | % (w/w) | | |
| FOL-005 | 1.53 | 1.53 | - |
| Sucrose | 2.97 | - | - |
| Sorbitan laurate | 4.00 | 2.00 | 4.00 |
| Isopropyl myristate | 91.50 | 96.47 | 96.00 |
| Total (%) | 100.00 | 100.00 | 100.00 |

| **Particle size, µm** | | | |
|---|---|---|---|
| D[v, 0.1] | 2.4 | 2.7 | n.a. |
| D[v, 0.5] | 8.4 | 7.2 | n.a. |
| D[v, 0.9] | 25.8 | 15.6 | n.a. |

**Table 12b. Compositions tested ex vivo in experiment 2.**

| **Batch** | FOL-005/sucrose suspension | FOL-005/sucrose formulation | Placebo |
|---|---|---|---|
| **Ingredient** | % (w/w) | | |
| FOL-005: sucrose | 4.52 | 1.56 | - |
| particles (contain 28% (w/w) FOL-005) | | | |
| FOL-005 Na-salt | - | - | - |
| Sorbitan laurate | 4.02 | 4.01 | 4.03 |
| Isopropyl myristate (dried) | 91.46 | 41.53 | 50.01 |
| Petrolatum | - | 49.91 | 42.93 |
| Glyceryl behenate | - | 2.99 | 3.03 |
| Total (%) | 100.00 | 100.00 | 100.00 |

### Skin membranes

Pig ear full thickness membranes were prepared in the following way:
The pig ears were rinsed with lukewarm water to remove dirt, blood and wax. Dry the ears using Kleenex and remove the bristles with a beard trimmer. Dermatome the inner ear skin into a thickness of approximately 600-700 µm and punch out membranes from the dermatomed skin pieces. The thickness of the skin membranes is determined using a micrometer. The skin membranes should be prepared the day before the ex *vivo* experiment and should be stored, covered with aluminium foil, in a refrigerator until they are used. The skin membranes are put in the diffusion cell equipment and allowed to hydrate for one hour at the chosen temperature for the experiment. After one hour of hydration 100 mg per cell of each formulation was applied. Massage of the formulation was performed using a small metallic spoon, tops "dressed" in parafilm and glass rods. At end of the experiment the skin samples were cleaned using tops and a solvent/fluid to remove access formulation. Water free propylene glycol was used as the solubility of FOL-005 is limited in propylene glycol (0.16 mg/ml in propylene glycol at room temperature) and it doesn't interact with sebum in the same way as non-polar solvents do.

### Ex vivo experimental design

The *in vitro* drug penetration experiment was performed in the following way: A 9 cell Frans cell equipment, Crown Glass Company, Inc., was used and, the volume of the cells were about 7 ml. Full thickness skin membranes were used for 48h experiment time with sampling at the end of the experiment. The temperature was 32°C in the Franz cells. Administration was performed two times, at 0 and 24 h. At each administration time, 100 mg of formulation was applied and gentle massage of the tissue was performed for 3 min using a glass rod. At termination of the experiment the skin samples are saved for analysis. In Table 12a and 12b the compositions for the formulations used in the ex *vivo* drug penetration experiments are given.

**Table 13a. Experimental design of the ex vivo drug penetration experiment number 1.**

| | **Cell 1-3** | **Cell 4-6** | **Cell 7-9** |
|---|---|---|---|
| **Membrane** | Full-thickness pig ear skin | Full-thickness pig ear skin | Full-thickness pig ear skin |
| **Receptor solution** | PBS pH 7.4 | PBS pH 7.4 | PBS pH 7.4 |
| **Formulation** Batch no. | FOL-005/sucrose suspension ISM 17209 | FOL-005/Na suspension ISM17216 | Placebo ISM17213 |
| **Dose, at 0 and 24 hours** | 100 mg | 100 mg | 100 mg |
| **Sampling (h)** | 48 | 48 | 24/48 |
| **Administration (h)** | 0 and 24 | 0 and 24 | 0 and 24 |
| **Experimental** time (h) | **48** | **48** | **48** |

**Table 13b. Experimental design of the ex vivo drug penetration experiment number 2.**

| | **Cell 1-3** | **Cell 4-6** | **Cell 8** |
|---|---|---|---|
| **Membrane** | Full-thickness pig ear skin | Full-thickness pig ear skin | Full-thickness pig ear skin |
| **Receptor solution** | PBS pH 7.4 | PBS pH 7.4 | PBS pH 7.4 |
| **Formulation Batch no.** | FOL-005/sucrose suspension ISM 18097 | FOL-005/sucrose formulation ISM 18098 | Placebo ISM 18099 |
| **Dose, at 0 and 24 hours** | 100 mg | 100 mg | 100 mg |
| **Sampling (h)** | 48 | 48 | 48 |
| **Administration (h)** | 0 and 24 | 0 and 24 | 0 and 24 |
| **Experimental** time (h) | **48** | **48** | **48** |

At termination of the experiment the skin membranes were cleaned using tops and fluid. Each cell was washed 5 times with propylene glycol. The retrieved material was collected in vials. The membranes were removed from the equipment and snap frozen.

### Maldi Mass Spectrometry Imaging (MSI)

Samples from the *ex vivo* experiment were analysed using MALDI mass spectrometry imaging. An analytical method was developed and the FOL-005 content and its distribution in the skin were studied.

Assessment and optimization of the detection of FOL-005 in the treated pig inner ear skin samples by MALDI FTICR MS Imaging was performed. Four different MALDI matrices (2,5-Dihydroxylbenzoic acid, α-Cyano-4-Hydroxycinnamic acid, 9-Aminoacridine and 1,5- Diaminoaphtalene) were evaluated and solvent optimization were performed for the optimal matrix.

Mass spectrometry imaging analysis was performed on pig inner ear skin biopsy samples by evaluating the frozen treated skin samples (n=1 per treated skin sample) and placebo sample (n=1 per placebo skin sample) using the developed MALDI-FTICR. In brief the frozen skin samples were sectioned in the hair follicles plane and one section per sample was analyzed by 7T-MALDI-FTICR imaging to determine the bio-distribution of FOL-005. Adjacent sections were stained with hematoxylin and eosin and were combined with the molecular distribution of FOL-005 to confirm the specific localization of the compound. The compound concentration per histological region was calculated by quantitative mass spectrometry imaging (QMSI) based on the generated MALDI images.

### Results

### Analysis of applied formulations

The formulations applied were analysed and the analysis was performed by high-pressure liquid chromatography (RP-HPLC) and ultra violet detection (UV). The compounds were monitored at 220 nm. The unidentified related substances were quantified as % relative area. FOL-005 salt (sodium) was used as external standard.

### Experiment number 1

The content of FOL-005 was found to be 1.33 % (w/w) in ISM17209 and 1.13 % (w/w) in ISM17216. The sum of related substances was 3.30 % in ISM17209 and 3.39 % in ISM17216, respectively. The measured particles size of the two formulations was about the same with an average size of 7.2 µm and 8.4 µm for FOL-005/sodium and FOL-005/sucrose particles, respectively.

### Experiment number 2

The content of FOL-005 were found to be 1.35 % (w/w) in ISM18097 and 0.47 % (w/w) in ISM18098. The sum of related substances was 1.65 % in ISM18097 and1.87 % in ISM 18098. The measured particles size of the two formulations was about the same with an average size of 7.2 µm and 8.4 µm for FOL-005/sodium and FOL-005/sucrose particles, respectively.

### Ex vivo experiments

The preferred MALDI matrix were DHB 40 mg/mL methanol/water and 0.1% TFA (v/v). The detection of FOL-005 in treated skin sections was also confirmed while no interfering peaks from the control tissue was detected.

### Experiment number 1

FOL-005 was detected in epidermis and dermis both in samples treated with FOL-005/sucrose suspension and FOL-005/Na suspension. Furthermore, FOL-005 was detected in a hair follicle of treated skin.

FOL-005 was detected in each treated skin while no signal was observed in the placebo treated sample. FOL-005 was mainly detected in epidermis with a decreasing gradient of FOL-005 from epidermis to dermis. This indicates that FOL-005 is transported by a transepidermal transport pathway in this experiment. However, some indications on a transfollicular penetration could also be observed with some hair follicles having a larger signal intensity than the surrounding dermis.

A strong heterogeneity between molecular distributions and quantification was observed in both FOL-005/sucrose and FOL-005/Na treated samples. Penetration depth of FOL-005 into each tissue were calculated as the maximum distance from the surface in which FOL-005 was detected.

Furthermore, the concentration of FOL-005 in the tissues was registered for epidermis, dermis and as the global concentration. In Table 14 the penetration depth of FOL-005 and concentrations in the tissues are shown. Despite the heterogenicity and large variability, a slightly larger FOL-005 accumulation could be seen in FOL-005/sucrose treated skin (71.4 ± 34.4 µg/g) compared to FOL-005/Na treated skin (35.7 ± 24.1 µg/g). However, no difference in the penetration depth between FOL-005/sucrose suspension and FOL-005/Na suspension could be detected.

**Table 14. Quantification of FOL-005 in the treated skin samples.**

| **Formulatio n** | **Penetratio n depth (mm)** | **Average penetratio n depth (mm)** | **Conc. Epidermi s (µg/g)** | **Conc. Dermi s (µg/g)** | **Conc. Globa l (µg/g)** | **Averag e conc. Dermis (µg/g)** |
|---|---|---|---|---|---|---|
| FOL-005/sucrose suspension | 1.59 | 1.10 ± 0.34 | 308.5 | 97.5 | 105.5 | 71.4± 34.4 |
| | 0.90 | | 56.9 | 22.8 | 26.9 | |
| | 0.83 | | 165.7 | 93.9 | 62.7 | |
| FOL-005/Na suspension | 1.53 | 0.97 ± 0.42 | 330.3 | 69.4 | 70.8 | 35.7± 24.1 |
| | 0.83 | | 30.3 | 14.7 | 16.0 | |
| | 0.54 | | 66.0 | 23.1 | 28.0 | |
| Placebo | nd | | nd | nd | nd | nd |

Quantification of FOL-005 in hair follicles was determined and results are presented in Table 15. The percentage of hair follicles presenting FOL-005 were larger for skin samples treated with FOL-005/sucrose suspension (56%) compared to skin samples treated with FOL-005/Na suspension (21%). It appeared that the depth of hair follicles presenting FOL-005 detection was larger in skin biopsies treated with sucrose suspension (0.48 ± 0.46 mm) than in skin biopsies treated with Na salt suspension (0.15 ± 0.16 mm).

**Table 15. Quantification of FOL-005 in the hair follicles.**

| **Formulation** | **Number of hair follicles analysed** | **Percentage of hair follicles presenting FOL-005 detection** | **Average depth of hair follicles presenting FOL-005 detection (mm)** | **Average concentration of FOL-005 in hair follicles (µg/g of tissue)*** |
|---|---|---|---|---|
| FOL-005/sucrose suspension | 18 | 56% | 0.48 ± 0.46 | 45.5 ± 76.8 |
| FOL-005/Na suspension | 28 | 21% | 0.15 ± 0.16 | 64.6 ± 185.1 |

| | | | | |
|---|---|---|---|---|
| * "nd" values were considered as "0" for the average concentration calculation | | | | |

The results indicate that the FOL-005/sucrose particle formulation have superior penetration properties compared to the FOL-005/sucrose particle formulation. The observed differences could be an effect of the difference in particle composition where one of the applied formulations contains particles with FOL-005 and sucrose while the particles in the other formulation only contains FOL-005. The measured particles size of the two formulations are about the same with an average size of 7.2 µm and 8.4 µm for FOL-005/sodium and FOL-005/sucrose particles, respectively. However, there are other differences between the formulations which could have an effect. The amount of Sorbitan laurate differs between the formulations where the formulation with FOL-005/sucrose particles contains 4% Sorbitan laurate and the formulation with FOL-005/sodium particles contains 2 % Sorbitan laurate .

### Experiment number 2

FOL-005 was weakly and heterogeneously detected in the epidermis and/or in the deep dermis of the tissues treated with formulations 18098. A more intense detection was observed in the pig ear samples treated with formulation 18097 with similar distribution in epidermis and deep dermis. Moreover, a passive diffusion from the epidermis to the dermis was observed. The dermis penetration was quantified at about 530 µm (high biological variability calculated at 51.3%).

A trans follicular parthway was particularly highlighted in some of the tissues. In these tissues, global concentrations of FOL-005 were quantified at 144.0 µg/g of tissue with a low biological variability calculated at 8.1%.

| **Formulati on** | **Penetrati on depth (µm)** | **Average penetratio n depth (µm)** | **Conc. Epidermi s (µg/g)** | **Conc. Dermi s (µg/g)** | **Conc. Deep Dermi s (µg/g)** | **Hair follicl e** | **Glob al (µg/g)** |
|---|---|---|---|---|---|---|---|
| FOL-005/sucros e suspension ISM 18097 | 250 | | 194..5 | 91.2 | 234.4 | BLOQ | 156.9 |
| | 800 | 533 | 422.9 | 144.5 | 86.8 | 119.1 | 140.9 |
| | 55 | | 523.4 | 104.9 | 88.7 | BLOQ | 134.3 |
| | | | | | | | Mean |
| | | | | | | | 144.0 |
| FOL-005/sucros e formulation ISM 18098 | 50 | 75 | 55.5 | BLOQ | 86.2 | BLOQ | 58.6 |
| | 100 | | 60.5 | BLOQ | | BLOQ | |
| | | | BLOQ | BLOQ | BLOQ | BLOQ | |
| | | | | | BLOQ | | |
| Placebo ISM 18099 | na | na | na | na | na | na | na |

### BLOQ Below Limit of Quantification = 53.7 µg/g

In Figure 4 overlays between Hematoxylin and Eosin adjacent section and FOL-005 molecular distribution can be seen for tissue samples treated with FOL-005/sucrose formulation.

### Conclusion

### Experiment number 1

FOL-005 were successfully transported into the tissue. A difference was detected between tissue treated with FOL-005/sucrose particles and tissue treated with FOL-005/sodium particles. Accumulation of FOL-005 was larger in tissues treated with FOL-005/sucrose compared to tissue treated with FOL-005/sodium particles. The average dermal concentration of FOL-005 in the skin was 71.4 ± 34.4 µg/g and 35.7 ± 24.1 µg/g for tissue treated with FOL-005/sucrose and FOL-005/sodium particles respectively. FOL005 was found in follicles in all the treated tissues. A larger fraction of the follicles in the tissues were found to contain FOL-005 in tissue treated with FOL-005/sucrose particle (56%) compared to tissue treated with FOL-005/sodium particles (21%). Similarly, the average depth of hair follicles presenting FOL-005 were larger in tissue treated with FOL-005/sucrose particle (0.48 ± 0.46 mm) compared to tissue treated with FOL-005/sodium particles (0.15 ± 0.16 mm). The results indicate that FOL-005/sucrose particles could have superior penetration properties compared to FOL-005/sodium particles.

### Experiment number 2

FOL-005 was successfully transported into the tissue. A difference was detected between tissue treated with FOL-005/sucrose formulation and tissue treated with FOL-005/sucrose suspension. Accumulation of FOL-005 was larger in tissues treated with FOL-005/sucrose suspension compared to tissue treated with FOL-005/sucrose formulation. This is at least partly explained by the lower FOL-005 concentration in the formulation as compared to suspension.

A passive diffusion from the epidermis to the dermis was observed. A trans follicular parthway was particularly highlighted in some of the tissues.

Data is shown in Figure 4.

### Example 6. In vivo testing

The present study was conducted to evaluate the hair growth promotion efficacy of FOL-005 in C57BL/6 mouse model by topical route of administration. Male C57BL/6 mice in stable telogen phase (resting phase) of hair growth cycle were used. The hair on the dorsal back of the animals was clipped and the area was treated with FOL-005 topical formulation, placebo formulation or commercially available Minoxidil 5%.

### Materials and Methods

Three formulations of FOL-005 (High dose- 0.5 %, Medium dose- 0.05 % and Low dose- 0.005 %) along with placebo were screened for hair growth promotion efficacy by applying 50 µL/cm2 on the dorsal clipped skin of animals in the respective groups. The application regimen was total of 4 weeks (5 days/week) followed by 1 week of observation period. During the study, all the animals were observed for skin color change from pink skin (telogen) to black skin (anagen) and appearance of new hair regrowth. After completion of observation period, it was observed that FOL-005 formulation led to hair growth promotion efficacy in a dose response manner. A faster anagen induction was observed in High dose (3/7 animals) followed by Medium dose (2/7 animals) and in Low dose (1/7 animal). Hair growth was observed in high dose (3/7 animals) and in medium dose (1/7 animals). No hair growth was observed in low dose of the formulation. Visual melanogenesis was also observed in the peeled skin of 3/7 animals, 2/7 animals and 1/7 animal in the High, Medium and Low dose respectively.

Minoxidil solution (5%) was used as a control and was applied topically (50 µL/cm2) on the dorsal clipped skin of the mice. The application regimen was twice daily application for total of 4 weeks followed by 1 week of observation period. Anagen induction followed by hair growth was observed in 4/5 animals in this group. Visual melanogenesis was also observed in the peeled skin of 4/5 animals.

### Conclusion

All the three topical formulations of FOL-005 (High dose, Medium dose and Low dose) demonstrated hair growth promotion efficacy in a dose dependent manner with highest hair growth promotion efficacy in High dose (0.5 %) followed by Medium dose (0.05 %) and then in Low dose (0.005 %).

Data is shown in Figure 5.

### Example 7. Method of manufacturing

In brief the manufacturing process is described as follows:
FOL-005 Sodium salt was freeze dried together with sucrose. The freeze-dried particles were mixed with dried Isopropyl myristate and Sorbitan laurate and then a ball milling step was performed.

The three excipients (Sorbitan laurate, Glyceryl behenate, Petrolatum) were added to the reactor at ambient temperature. The reactor was fitted with a stirrer. The excipients were mixed at room temperature until a homogenous viscous gel was formed. The reactor was then heated to 75°C under stirring and then cooled to room temperature. Thereafter the ball milled FOL-005/sucrose - Isopropyl myristate suspension was added to the reactor and the mixture was stirred until a homogeneous mixture is obtained.

### Example 8. Compositions comprising pepetides

In this study, compositions comprising a number of representative peptides (see table 16) were manufactured. Further, the compositions were stored for 4 weeks at 20°C to investigate the stability of the peptides in the compositions.

**Table 16. Peptides.**

| SEQ ID NO | Number of amino acids | Amino acid sequence | Name |
|---|---|---|---|
| 188 | 3 | GHK | GHK |
| 186 | 9 | CYIQNCPLG | Oxytocin |
| 69 | 15 | VDVPNGDISLAYGLR | FOL-004 |
| 136 | 16 | KPLAEIDSIELSYGIK | FOL-014 |
| 185 | 25 | GKYGFYTHVFRLKKWIQKVIDQFGE | FOL-199 |
| 187 | 37 | LLGDFFRKSKEKIGKEFKRIVQRIKDFLRNLVPRTES | LL-37 |

### Manufacture

Each peptide was lyophilized together with sucrose in a w/w-ratio 2:1 (Sucrose/Peptide) according to standard lyophilization methods.

Span20 was mixed with IPM to obtain a 4% (w/w) homogenous solution.

The peptide-sucrose was added to the Span20-IPM mixture to obtain a 0.4% suspension with regard to the peptide content. Using a standardized bead, wet-milling method the peptide-sucrose particles were micronized in the Span20-IPM suspension.

An ointment base was produced by mixing 89.5% Petrolatum, 4.2% Span20 and 6.3 % Glyceryl behenate (w/w %). The mixture was heated to 75°C during stirring. The stirring was continued until glyceryl behenate was dissolved. Then the ointment base was cooled to room temperature under gentle stirring.

When the ointment base had been cooled to room temperature, the ointment base was added to the separate peptide suspensions to obtain 0.2% ointments with regard to peptide concentration. The mixture was stirred at ambient temperature for 2 hours. The total composition is given in table 17.

**Table 17. Total composition.**

| **Component** | **Amount (wt%)** |
|---|---|
| Peptide | 0.20 |
| Sucrose | 0.40 |
| Span20 | 4.0 |
| Isopropyl myristate (IPM) | 50.0 |
| Petrolatum | 42.4 |
| Glycerol behenate | 3.0 |

### Stability study

Samples of the separate formulations were stored at 20°C. Further, samples of PBS solutions of oxytocin and FOL-004, respectively, were prepared and stored at 20°C.

Analysis of peptide purity in ointment and PBS solutions was performed using standard HPLC-UV-DA methods when the samples were freshly prepared (time 0) and after 1, 2 and 4 weeks. Data is shown in Figure 6.

### Sequences

| **SEQ ID NO** | **Sequence** | **Notes** |
|---|---|---|
| **1** | VDTYDGDISVVYGLR | FOL-005 |
| **2** | VDTYDGDISVVYGLS | |
| **3** | VDTYDGDISVVYGL | FOL-025 |
| **4** | DTYDGDISVVYGLR | |
| **5** | TYDGDISVVYGLRS | |
| **6** | VDTYDGDISVVYG | FOL-024 |
| **7** | DTYDGDISVVYGL | |
| **8** | TYDGDISVVYGLR | |
| **9** | YDGDISVVYGLRS | |
| **10** | VDTYDGDISVVY | |
| **11** | DTYDGDISVVYG | |
| **12** | TYDGDISVVYGL | |
| **13** | YDGDISVVYGLR | |
| **14** | DGDISVVYGLRS | |
| **15** | VDTYDGDISVV | |
| **16** | DTYDGDISVVY | |
| **17** | TYDGDISVVYG | |
| **18** | YDGDISVVYGL | |
| **19** | DGDISVVYGLR | |
| **20** | GDISVVYGLRS | |
| **21** | VDTYDGDISV | |
| **22** | DTYDGDISVV | |
| **23** | TYDGDISVVY | |
| **24** | YDGDISVVYG | |
| **25** | DGDISVVYGL | |
| **26** | GDISVVYGLR | FOL-009h |
| **27** | DISVVYGLRS | |
| **28** | VDTYDGDIS | FOL-019h |
| **29** | DTYDGDISV | |
| **30** | TYDGDISVV | |
| **31** | YDGDISVVY | |
| **32** | DGDISVVYG | |
| **33** | GDISVVYGL | |
| **34** | DISVVYGLR | |
| **35** | ISVVYGLRS | |
| **36** | VDTYDGDI | |
| **37** | DTYDGDIS | |
| **38** | TYDGDISV | |
| **39** | YDGDISVV | |
| **40** | DGDISVVY | |
| **41** | GDISVVYG | |
| **42** | DISVVYGL | |
| **43** | ISVVYGLR | |
| **44** | VDTYDGD | |
| **45** | DTYDGDI | |
| **46** | TYDGDIS | |
| **47** | YDGDISV | |
| **48** | DGDISVV | |
| **49** | GDISVVY | |
| **50** | DISVVYG | |
| **51** | ISVVYGL | |
| **52** | DTYDGD | |
| **53** | TYDGDI | |
| **54** | YDGDIS | |
| **55** | DGDISV | |
| **56** | GDISVV | |
| **57** | DISVVY | |
| **58** | ISVVYG | |
| **59** | TYDGD | |
| **60** | YDGDI | |
| **61** | DGDIS | |
| **62** | GDISV | |
| **63** | DISVV | |
| **64** | ISVVY | |
| **65** | SVVYG | |
| **66** | MRIAVICFCLLGITCAIPVKQADSGSSEEKQLY NKYPDAVATWLNPDPSQKQNLLAPQTLPSK SNESHDHMDDMDDEDDDDHVDSQDSIDSN DSDDVDDTDDSHQSDESHHSDESDELVTDF PTDLPATEVFTPVVPTVDTYDGRGDSVVYGL RSKSKKFRRPDIQYPDATDEDITSHMESEEL NGAYKAIPVAQDLNAPSDWDSRGKDSYETS QLDDQSAETHSHKQSRLYKRKANDESNEHS DVIDSQELSKVSREFHSHEFHSHEDMLVVDP KSKEEDKHLKFRISHELDSASSEVN | Wildtype human osteopontin, i.e. GenBank: AAA59974.1 |
| **67** | VDTYDGRGDSVVYGLR | FOL-002 |
| **68** | VDZ₃Z₄Z₅GZ₇Z₉SZ₁₀Z₁₁YGLR | Z₃ is T or V; |
| | | Z₄ is Y or P; |
| | | Z₅ is D or N; |
| | | Z₇ is D or G; |
| | | Z₈ is I or G; |
| | | Z₁₀ is V or L; |
| | | Z₁₁ is V or A |
| **69** | VDVPNGDISLAYGLR | FOL-004 |
| **70** | DVPNGDISLAYGLRS | |
| **71** | VDVPNGDISLAYGL | FOL-016 |
| **72** | DVPNGDISLAYGLR | FOL-007 |
| **73** | VPNGDISLAYGLRS | |
| **74** | VDVPNGDISLAYG | FOL-017 |
| **75** | DVPNGDISLAYGL | |
| **76** | VPNGDISLAYGLR | |
| **77** | PNGDISLAYGLRS | |
| **78** | VDVPNGDISLAY | |
| **79** | DVPNGDISLAYG | |
| **80** | VPNGDISLAYGL | |
| **81** | PNGDISLAYGLR | FOL-008 |
| **82** | NGDISLAYGLRS | |
| **83** | VDVPNGDISLA | FOL-018 |
| **84** | DVPNGDISLAY | |
| **85** | VPNGDISLAYG | |
| **86** | PNGDISLAYGL | |
| **87** | NGDISLAYGLR | |
| **88** | GDISLAYGLRS | |
| **89** | VDVPNGDISL | |
| **90** | DVPNGDISLA | |
| **91** | VPNGDISLAY | |
| **92** | PNGDISLAYG | |
| **93** | NGDISLAYGL | |
| **94** | GDISLAYGLR | FOL-009 |
| **95** | DISLAYGLRS | |
| **96** | VDVPNGDIS | FOL-019 |
| **97** | DVPNGDISL | |
| **98** | VPNGDISLA | |
| **99** | PNGDISLAY | |
| **100** | NGDISLAYG | |
| **101** | GDISLAYGL | |
| **102** | DISLAYGLR | |
| **103** | ISLAYGLRS | |
| **104** | VDVPNGDI | |
| **105** | DVPNGDIS | |
| **106** | VPNGDISL | |
| **107** | PNGDISLA | |
| **108** | NGDISLAY | |
| **109** | GDISLAYG | |
| **110** | DISLAYGL | |
| **111** | ISLAYGLR | |
| **112** | VDVPNGD | |
| **113** | DVPNGDI | |
| **114** | VPNGDIS | |
| **115** | PNGDISL | |
| **116** | NGDISLA | |
| **117** | GDISLAY | |
| **118** | DISLAYG | |
| **119** | ISLAYGL | |
| **120** | DVPNGD | |
| **121** | VPNGDI | |
| **122** | PNGDIS | |
| **123** | NGDISL | |
| **124** | GDISLA | |
| **125** | DISLAY | |
| **126** | ISLAYG | |
| **127** | VPNGD | |
| **128** | PNGDI | |
| **129** | NGDIS | |
| **130** | GDISL | |
| **131** | DISLA | |
| **132** | ISLAY | |
| **133** | SLAYG | |
| **134** | MRLAVICFCLFGIASSLPVKVTDSGSSEEKLY SLHPDPIATWLVPDPSQKQNLLAPQNAVSSE EKDDFKQETLPSNSNESHDHMDDDDDDDD | Wildtype murine osteopontin, *i.e.* NCBI Reference |
| | DDGDHAESEDSVDSDESDESHHSDESDETV TASTQADTFTPIVPTVDVPNGRGDSLAYGLR SKSRSFQVSDEQYPDATDEDLTSHMKSGES KESLDVIPVAQLLSMPSDQDNNGKGSHESS QLDEPSLETHRLEHSKESQESADQSDVIDSQ ASSKASLEHQSHKFHSHKDKLVLDPKSKEDD RYLKFRISHELESSSSEVN | Sequence: NP_001191162.1 |
| **135** | VDVPNGRGDSLAYGLR | FOL-001 |
| **136** | KPLAEIDSIELSYGIK | FOL-014 |
| **137** | GDPNDGRGDSVVYGLR | FOL-003 |
| **138** | VDTYDGGISVVYGLR | FOL-026 |
| **139** | VDTYDGDGSVVYGLR | FOL-027 |
| **140** | KX₂LAX₅X₆X₇X₈IX₁₀LX₁₂YGIK | X₂ is C, P or G; |
| | | X₅ is E or G; |
| | | X₆ is C, D or I; |
| | | X₇ is D, I, S or G; |
| | | X₈ is S, D or G; |
| | | X₁₀ is E or G; |
| | | X₁₂ is S or T; |
| **141** | KCLAECDSIELSYGIK (Cyclic) | FOL-032 |
| **142** | CLAEIDSC (Cyclic) | FOL-033 |
| **143** | CFKPLAEIDSIECSYGIK (Cyclic) | FOL-036 |
| **144** | KPLAEDISIELSYGIK | FOL-037 |
| **145** | KPLAEISDIELSYGIK | FOL-038 |
| **146** | KPLAEIGDIELSYGIK | FOL-039 |
| **147** | KPLAEGDIELSYGIK | FOL-040 |
| **148** | KPLAEIELSYGIK | FOL-041 |
| **149** | KPLAEIDSIELTYGIK | FOL-042 |
| **150** | KPLAEIDGIELSYGIK | FOL-043 |
| **151** | KPLAEIDGIELTYGIK | FOL-044 |
| **152** | KPLAEIGSIELSYGIK | FOL-045 |
| **153** | KGLAEIDSIELSYGIK | FOL-046 |
| **154** | KPLAGIDSIGLSYGIK | FOL-047 |
| **155** | Cyclic KCLAEIDSCELSYGIK | FOL-034 |
| **156** | Cyclic CFKPLAEIDSIEC | FOL-035 |
| **157** | VDVPEGDISLAYGLR | FOL-010 |
| **158** | LDGLVRAYDNISPVG | FOL-015 |
| **159** | GDPNGDISVVYGLR | FOL-006 |
| **160** | VDVPNGDISLAYRLR | FOL-011 |
| **161** | VDVPEGDISLAYRLR | FOL-012 |
| **162** | KX₂LAX₅X₆X₇X₈IX₁₀LSYGIK | X₂ is C, P or G; |
| | | X₅ is E or G; |
| | | X₆ is C, I or absent; |
| | | X₇ is D, G or absent; |
| | | X₈ is S, G or absent; |
| | | X₁₀ is E or G; |
| **163** | KX₂LAX₅IX₁₀LSYGIK | X₂ is C, P or G; |
| | | X₅ is E or G; |
| | | X₁₀ is E or G. |
| **164** | VDVPZ₅GDISLAYZ₁₃LR | Z₅ is E or N; |
| | | Z₁₃ is R or G. |
| **165** | VDTYDGZ₇Z₈SVVYGLR | Z₇ is D or G; |
| | | Z₈ is I or G. |
| **166** | GDPNZ₅Z₆Z₇Z₈Z₉SVVYGLR | Z₅ is D or G; |
| | | Z₆ is D or G |
| | | Z₇ is I or R; |
| | | Z₈ is G or absent; |
| | | Z₉ is D or absent. |
| **167** | VZ₂TYDGDISVVYGLR | Z₂ is beta D |
| | | FOL-005 (2betaAsp) |
| **168** | VDTY Z₅GDISVVYGLR | Z₅ is beta D |
| | | FOL-005 (5betaAsp) |
| **169** | VDTYDG Z₇ISVVYGLR | FOL-005 (7betaAsp) |
| | | Z₇ is beta D |
| **170** | LAEIDSIELSYGIK | |
| **171** | AEIDSIELSYGIK | FOL-056 |
| **172** | EIDSIELSYGIK | FOL-057 |
| **173** | IDSIELSYGIK | FOL-058 |
| **174** | DSIELSYGIK | FOL-059 |
| **175** | SIELSYGIK | FOL-060 |
| **176** | IELSYGIK | |
| **177** | X₁₀LX₁₂YGIK | X₁₀ is E or G; |
| | | X₁₂ is S or T; |
| **178** | Z₇Z₈SZ₁₀Z₁₁YGLR | Z₇ is D or G; |
| | | Z₈ is I or G; |
| | | Z₁₀ is V or L; |
| | | Z₁₁ is V or A; |
| **179** | KPLAEIDSIELSYGI | |
| **180** | KPLAEIDSIELSYG | |
| **181** | KPLAEIDSIELSY | |
| **182** | KPLAEIDSIELS | |
| **183** | KPLAEIDSIEL | |
| **184** | KPLAEIDSIE | |
| **185** | GKYGFYTHVFRLKKWIQKVIDQFGE | FOL-199 |
| **186** | CYIQNCPLG | Oxytocin |
| **187** | LLGDFFRKSKEKIGKEFKRIVQRIKDFLRNLVP RTES | LL-37 |
| **188** | GHK | GHK |

## Claims

1. A composition comprising:
i) 0.01 to 2 wt% peptide or peptide derivative;
ii) 0.01 to 4 wt% saccharide or modified saccharide;
iii) 35 to 50 wt% petrolatum; and
iv) 40 to 60 wt% isopropyl myristate,
with the proviso that the sum of the components of the total composition does not exceed 100 wt%.

2. The composition according to claim 1, wherein the composition further comprises 1 to 8 wt% thickener, such as glyceryl behenate or carnauba wax.

3. The composition according to any one of the preceding claims, wherein the composition further comprises 2 to 10 wt% surfactant, such as sorbitan laurate.

4. The composition according to any one of the preceding claims, wherein the saccharide or modified saccharide is selected from the group consisting of sucrose, mannitol and glucose.

5. The composition according to any one of the preceding claims, wherein the composition comprises or consists of:
a. 0.01 to 2 wt% peptide or peptide derivative;
b. 0.01 to 4 wt% sucrose, mannitol or glucose;
c. 1 to 8 wt% glyceryl behenate or carnauba wax;
d. 35 to 45 wt% petrolatum;
e. 40 to 60 wt% isopropyl myristate; and
f. 2 to 10 wt% surfactant, such as sorbitan laurate.

6. The composition according to any one of the preceding claims, wherein the composition comprises or consists of:
i) 0.01 to 2 wt% peptide or peptide derivative;
ii) 0.01 to 4 wt% sucrose;
iii) 3 wt% glyceryl behenate;
iv) 42.4 wt% petrolatum;
v) 50 wt% isopropyl myristate; and
vi) 4 wt% sorbitan laurate,
such as wherein the composition comprises or consists of:
a. 0.2 wt% peptide or peptide derivative;
b. 0.4 wt% sucrose;
c. 3 wt% glyceryl behenate;
d. 42.4 wt% petrolatum;
e. 50 wt% isopropyl myristate; and
f. 4 wt% sorbitan laurate.

7. The composition according to any one of the preceding claims, wherein the composition comprises particles comprising or consisting of the peptide or peptide derivative and the saccharide or modified saccharide, wherein at least 50% of the particles have an average particle diameter of between 0.1 and 50 µm, for example between 0.1 and 15 µm, such as between 0.1 and 10 µm, such as between 0.1 and 2 µm.

8. The composition according to any one of the preceding claims, wherein the peptide or peptide derivative comprises or consists of from 3 to 50 amino acid residues, such as from 3 to 40 amino acid residues, such as 5 to 30 amino acid residues, such as 10 to 25 amino acid residues.

9. The composition according to any one of the preceding claims, wherein the peptide or peptide derivative comprises:
i) an amino acid sequence of the general formula:
VDTYDGZ₇Z₈SVVYGLR (SEQ ID NO: 165)
wherein:
Z₇ is D or G;
Z₈ is I or G;
ii) an amino acid sequence of the general formula:
KX₂LAX₅X₆X₇X₈IX₁₀LX₁₂YGIK (SEQ ID NO: 140)
wherein:
X₂ is C, P or G;
X₅ is E or G;
X₆ is C, D or I;
X₇ is D, I, S or G;
X₈ is S, D or G;
X₁₀ is E or G;
X₁₂ is S or T;
with the proviso that if X₁₂ is T, the peptide comprises no more than 25 amino acid residues; and
with the proviso that if X₂ is P, X₅ is E, X₆ is I, X₇ is D, X₈ is S, X₁₀ is E and X₁₂ is S, the peptide comprises no more than 85 amino acid residues;
iii) an amino acid sequence of the general formula:
X₁₀LX₁₂YGIK (SEQ ID NO: 177)
wherein:
X₁₀ is E or G;
X₁₂ is S or T;
with the proviso that if X₁₂ is T, the peptide comprises no more than 25 amino acids; and
with the proviso that if X₁₀ is E and X₁₂ is S, the peptide comprises no more than 85 amino acid residues;
iv) an amino acid sequence of the general formula:
VDVPZ₅GDISLAYZ₁₃LR (SEQ ID NO: 164)
wherein:
Z₅ is E or N;
Z₁₃ is R or G;
v) an amino acid sequence of the general formula:
VDTYDGZ₇Z₈SVVYGLR (SEQ ID NO: 165)
wherein:
Z₇ is D or G;
Z₈ is I or G;
vi) an amino acid sequence of the general formula:
GDPNZ₅Z₆Z₇Z₈Z₉SVVYGLR (SEQ ID NO: 166)
wherein:
Z₅ is D or G;
Z₆ is D or G
Z₇ is I or R;
Z₈ is G or absent;
Z₉ is D or absent;
vii) an amino acid sequence of the general formula:
KX₂LAX₅X₆X₇X₈IX₁₀LSYGIK (SEQ ID NO: 162)
wherein:
X₂ is C, P or G;
X₅ is E or G;
X₆ is C, I or absent;
X₇ is D, G or absent;
X₈ is S, G or absent;
X₁₀ is E or G;
viii) an amino acid sequence of the general formula:
KX₂LAX₅IX₁₀LSYGIK (SEQ ID NO: 163)
wherein:
X₂ is C, P or G;
X₅ is E or G;
X₁₀ is E or G;
i) an amino acid sequence of the general formula:
Z₇Z₈SZ₁₀Z₁₁YGLR (SEQ ID NO: 178)
wherein:
Z₇ is D or G;
Z₈ is I or G;
Z₁₀ is V or L;
Z₁₁ is V or A;
or
ix) an amino acid sequence of the general formula:
VDZ₃Z₄Z₅GZ₇Z₈SZ₁₀Z₁₁YGLR (SEQ ID NO: 68)
wherein:
Z₃ is T or V;
Z₄ is Y or P;
Z₅ is D or N;
Z₇ is D or G;
Z₈ is I or G;
Z₁₀ is V or L;
Z₁₁ is V or A.

10. The composition according to claim 1, wherein the peptide or peptide derivative comprises or consists of:
i) the amino acid sequence of VDTYDGDISVVYGLR (SEQ ID NO: 1; FOL-005) or a fragment or variant thereof;
ii) the amino acid sequence of KPLAEIDSIELSYGIK (SEQ ID NO: 136, FOL-014) or a fragment or variant thereof;
iii) the amino acid sequence of VDVPNGDISLAYGLR (SEQ ID NO: 69, FOL-004) or a fragment or variant thereof;
iv) an amino acid sequence selected from the group consisting of GDPNDGRGDSVVYGLR (SEQ ID NO: 137), VDTYDGGISVVYGLR (SEQ ID NO: 138), VDTYDGDGSVVYGLR (SEQ ID NO: 139). VDVPEGDISLAYGLR (SEQ ID NO: 157), LDGLVRAYDNISPVG (SEQ ID NO: 158), GDPNGDISVVYGLR (SEQ ID NO: 159), VDVPNGDISLAYRLR (SEQ ID NO: 160), VDVPEGDISLAYRLR (SEQ ID NO: 161), V(beta-D)TYDGDISVVYGLR (SEQ ID NO:167), VDTY(beta-D)GDISVVYGLR (SEQ ID NO: 168) and VDTYDG(beta-D)ISVVYGLR (SEQ ID NO:169);
v) an amino acid sequence selected from the group consisting of KCLAECDSIELSYGIK (SEQ ID NO: 141), CLAEIDSC (SEQ ID NO: 142), CFKPLAEIDSIECSYGIK (SEQ ID NO: 143), KPLAEDISIELSYGIK (SEQ ID NO: 145), KPLAEIGDIELSYGIK (SEQ ID NO: 146), KPLAEGDIELSYGIK (SEQ ID NO: 147), KPLAEIELSYGIK (SEQ ID NO: 148), KPLAEIDSIELTYGIK (SEQ ID NO: 149), KPLAEIDGIELSYGIK (SEQ ID NO: 150), KPLAEIDGIELTYGIK (SEQ ID NO: 151), KPLAEIGSIELSYGIK (SEQ ID NO: 152), KGLAEIDSIELSYGIK (SEQ ID NO: 153), KPLAGIDSIGLSYGIK (SEQ ID NO: 154), KCLAEIDSCELSYGIK (SEQ ID NO: 155) and CFKPLAEIDSIEC (SEQ ID NO: 156), or a variant or fragment thereof;
vi) an amino acid sequence selected from the group consisting of LAEIDSIELSYGIK (SEQ ID NO: 170), AEIDSIELSYGIK (SEQ ID NO: 171), EIDSIELSYGIK (SEQ ID NO: 172), IDSIELSYGIK (SEQ ID NO: 173), DSIELSYGIK (SEQ ID NO: 174), SIELSYGIK (SEQ ID NO: 175), IELSYGIK (SEQ ID NO: 176), or a variant or fragment thereof; or
vii) an amino acid sequence selected from the group consisting of KPLAEIDSIELSYGI (SEQ ID NO: 179), KPLAEIDSIELSYG (SEQ ID NO: 180), KPLAEIDSIELSY (SEQ ID NO: 181), KPLAEIDSIELS (SEQ ID NO: 182), KPLAEIDSIEL (SEQ ID NO: 183), KPLAEIDSIE (SEQ ID NO: 184), or a variant of fragment thereof;
or wherein the peptide or peptide derivative is selected from the group consisting of GHK (SEQ ID NO: 188), oxytocin (SEQ ID NO: 186), polymyxin B, LL37 (SEQ ID NO: 187), FOL-199 (SEQ ID NO: 185) and becaplermin;
or wherein the peptide or peptide derivative is an antimicrobial peptide or peptide derivative or an anti-inflammatory peptide or peptide derivative.

11. The composition according to any one of the preceding claims, wherein the peptide derivative is a peptide according to any one of the preceding claims, which is conjugated to a moiety, such as a moiety selected from the group consisting of polyethylene glycol (PEG), monosaccharides, fluorophores, chromophores, radioactive compounds, and cell-penetrating peptides; and/or wherein the peptide derivative is a peptide according to any one of the preceding claims, which is modified by being glycosylated or by PEGylation, amidation, esterification, acylation, acetylation and/or alkylation; and/or wherein the peptide derivative is a peptide according to any one of the preceding claims, which is fused to another polypeptide, such as a polypeptide selected from the group consisting of glutathione-S-transferase (GST) and protein A, or to a tag.

12. The composition according to any one of the preceding claims, wherein the composition is in the form of an ointment, a powder, a spray, a lotion, a gel, foam, a cream, a make-up or a shampoo.

13. The composition according to any one of the preceding claims for use as a medicament.

14. The composition according to any one of the preceding claims for use in the treatment or prevention of a disease or condition associated with hair loss or a dermatological condition, such as a dermatological condition selected from the group consisting of psoriasis, atopic dermatitis, eczema, precarcinogenic states and skin infections.

15. A method of manufacturing the composition according to any one of claims 1 to 12 comprising the following steps:
a) mixing the peptide with a saccharide;
b) freeze drying the mixture of a);
c) mixing b) with a lipid and a surfactant;
d) grinding the mixture of c); and
e) optionally mixing the mixture of d) with a lipid and a thickener.

## Patentansprüche

1. Zusammensetzung, welche Folgendes umfasst:
i) 0,01 bis 2 Gew.% Peptid oder Peptidderivat;
ii) 0,01 bis 4 Gew.% Saccharid oder modifiziertes Saccharid;
iii) 35 bis 50 Gew.% Vaseline; und
iv) 40 bis 60 Gew.% Isopropylmyristat,
mit der Maßgabe, dass die Summe der Komponenten der Gesamtzusammensetzung 100 Gew.% nicht übersteigt.

2. Zusammensetzung nach Anspruch 1, wobei die Zusammensetzung ferner 1 bis 8 Gew.% Verdickungsmittel, wie z. B. Glycerylbehenat oder Carnaubawachs, umfasst.

3. Zusammensetzung nach einem der vorhergehenden Ansprüche, wobei die Zusammensetzung ferner 2 bis 10 Gew.% Tensid, wie z. B. Sorbitanlaurat, umfasst.

4. Zusammensetzung nach einem der vorhergehenden Ansprüche, wobei das Saccharid oder das modifizierte Saccharid ausgewählt ist aus der Gruppe bestehend aus Saccharose, Mannitol und Glukose.

5. Zusammensetzung nach einem der vorhergehenden Ansprüche, wobei die Zusammensetzung Folgendes umfasst oder daraus besteht:
a. 0,01 bis 2 Gew.% Peptid oder Peptidderivat;
b. 0,01 bis 4 Gew.% Saccharose, Mannitol oder Glukose;
c. 1 bis 8 Gew.% Glycerylbehenat oder Carnaubawachs;
d. 35 bis 45 Gew.% Vaseline;
e. 40 bis 60 Gew.% Isopropylmyristat; und
f. 2 bis 10 Gew.% Tensid, wie z. B. Sorbitanlaurat.

6. Zusammensetzung nach einem der vorhergehenden Ansprüche, wobei die Zusammensetzung Folgendes umfasst oder daraus besteht:
i) 0,01 bis 2 Gew.% Peptid oder Peptidderivat;
ii) 0,01 bis 4 Gew.% Saccharose;
iii) 3 Gew.% Glycerylbehenat;
iv) 42,4 Gew.% Vaseline;
v) 50 Gew.% Isopropylmyristat; und
vi) 4 Gew.% Sorbitanlaurat,
wobei die Zusammensetzung beispielsweise Folgendes umfasst oder daraus besteht:
a. 0,2 Gew.% Peptid oder Peptidderivat;
b. 0,4 Gew.% Saccharose;
c. 3 Gew.% Glycerylbehenat;
d. 42,4 Gew.% Vaseline;
e. 50 Gew.% Isopropylmyristat; und
f. 4 Gew.% Sorbitanlaurat.

7. Zusammensetzung nach einem der vorhergehenden Ansprüche, wobei die Zusammensetzung Partikel umfasst, die das Peptid oder das Peptidderivat und das Saccharid oder das modifizierte Saccharid umfassen oder daraus bestehen, wobei mindestens 50 % der Partikel einen durchschnittlichen Partikeldurchmesser zwischen 0,1 und 50 µm aufweisen, zum Beispiel zwischen 0,1 und 15 pm, wie z. B. zwischen 0,1 und 10 pm, wie z. B. zwischen 0,1 und 2 pm.

8. Zusammensetzung nach einem der vorhergehenden Ansprüche, wobei das Peptid oder das Peptidderivat von 3 bis 50 Aminosäurereste umfasst oder daraus besteht, wie z. B. von 3 bis 40 Aminosäurereste, wie z. B. 5 bis 30 Aminosäurereste, wie z. B. 10 bis 25 Aminosäurereste.

9. Zusammensetzung nach einem der vorhergehenden Ansprüche, wobei das Peptid oder das Peptidderivat Folgendes umfasst:
i) eine Aminosäuresequenz mit der allgemeinen Formel:
VDTYDGZ₇Z₈SVVYGLR (SEQ ID NO: 165)
wobei:
Z₇ D oder G ist;
Z₈ I oder G ist;
ii) eine Aminosäuresequenz mit der allgemeinen Formel:
KX₂LAX₅X₆X₇X₈IX₁₀LX₁₂YGIK (SEQ ID NO: 140)
wobei:
X₂ C, P oder G ist;
X₅ E oder G ist;
X₆ C, D oder I ist;
X₇ D, I, S oder G ist;
X₈ S, D oder G ist;
X₁₀ E oder G ist;
X₁₂ S oder T ist;
mit der Maßgabe, dass, wenn X₁₂ T ist, das Peptid nicht mehr als 25 Aminosäurereste umfasst; und
mit der Maßgabe, dass, wenn X₂ P ist, X₅ E ist, X₆ I ist, X₇ D ist, X₈ S ist, X₁₀ E ist und X₁₂ S ist, das Peptid nicht mehr als 85 Aminosäurereste umfasst;
iii) eine Aminosäuresequenz mit der allgemeinen Formel:
X₁₀LX₁₂YGIK (SEQ ID NO: 177)
wobei:
X₁₀ E oder G ist;
X₁₂ S oder T ist;
mit der Maßgabe, dass, wenn X₁₂ T ist, das Peptid nicht mehr als 25 Aminosäuren umfasst; und
mit der Maßgabe, dass, wenn X₁₀ E ist und X₁₂ S ist, das Peptid nicht mehr als 85 Aminosäurereste umfasst;
iv) eine Aminosäuresequenz mit der allgemeinen Formel:
VDVPZ₅GDISLAYZ₁₃LR (SEQ ID NO: 164)
wobei:
Z₅ E oder N ist;
Z₁₃ R oder G ist;
v) eine Aminosäuresequenz mit der allgemeinen Formel:
VDTYDGZ₇Z₈SVVYGLR (SEQ ID NO: 165)
wobei:
Z₇ D oder G ist;
Z₈ I oder G ist;
vi) eine Aminosäuresequenz mit der allgemeinen Formel:
GDPNZ₅Z₆Z₇Z₈Z₉SVVYGLR (SEQ ID NO: 166)
wobei:
Z₅ D oder G ist;
Z₆ D oder G ist;
Z₇ I oder R ist;
Z₈ G ist oder fehlt;
Z₉ D ist oder fehlt;
vii) eine Aminosäuresequenz mit der allgemeinen Formel:
KX₂LAX₅X₆X₇X₈IX₁₀LSYGIK (SEQ ID NO: 162)
wobei:
X₂ C, P oder G ist;
X₅ E oder G ist;
X₆ C oder I ist oder fehlt;
X₇ D oder G ist oder fehlt;
X₈ S oder G ist oder fehlt;
X₁₀ E oder G ist;
viii) eine Aminosäuresequenz mit der allgemeinen Formel:
KX₂LAX₅IX₁₀LSYGIK (SEQ ID NO: 163)
wobei:
X₂ C, P oder G ist;
X₅ E oder G ist;
X₁₀ E oder G ist;
i) eine Aminosäuresequenz mit der allgemeinen Formel:
Z₇Z₈SZ₁₀Z₁₁YGLR (SEQ ID NO: 178)
wobei:
Z₇ D oder G ist;
Z₈ I oder G ist;
Z₁₀ V oder L ist;
Z₁₁ V oder A ist; oder
ix) eine Aminosäuresequenz mit der allgemeinen Formel:
VDZ₃Z₄Z₅GZ₇Z₈SZ₁₀Z₁₁YGLR (SEQ ID NO: 68)
wobei:
Z₃ T oder V ist;
Z₄ Y oder P ist;
Z₅ D oder N ist;
Z₇ D oder G ist;
Z₈ I oder G ist;
Z₁₀ V oder L ist;
Z₁₁ V oder A ist.

10. Zusammensetzung nach Anspruch 1, wobei das Peptid oder das Peptidderivat Folgendes umfasst oder daraus besteht:
i) die Aminosäuresequenz von VDTYDGDISVVYGLR (SEQ ID NO: 1; FOL-005) oder ein Fragment oder eine Variante davon;
ii) die Aminosäuresequenz von KPLAEIDSIELSYGIK (SEQ ID NO: 136, FOL-014) oder ein Fragment oder eine Variante davon;
iii) die Aminosäuresequenz von VDVPNGDISLAYGLR (SEQ ID NO: 69, FOL-004) oder ein Fragment oder eine Variante davon;
iv) eine Aminosäuresequenz ausgewählt aus der Gruppe bestehend aus GDPNDGRGDSVVYGLR (SEQ ID NO: 137), VDTYDGGISVVYGLR (SEQ ID NO: 138), VDTYDGDGSVVYGLR (SEQ ID NO: 139), VDVPEGDISLAYGLR (SEQ ID NO: 157), LDGLVRAYDNISPVG (SEQ ID NO: 158), GDPNGDISVVYGLR (SEQ ID NO: 159), VDVPNGDISLAYRLR (SEQ ID NO: 160), VDVPEGDISLAYRLR (SEQ ID NO: 161), V(beta-D)TYDGDISVVYGLR (SEQ ID NO: 167), VDTY(beta-D)GDISVVYGLR (SEQ ID NO: 168) und VDTYDG(beta-D)ISVVYGLR (SEQ ID NO: 169);
v) eine Aminosäuresequenz ausgewählt aus der Gruppe bestehend aus KCLAECDSIELSYGIK (SEQ ID NO: 141), CLAEIDSC (SEQ ID NO: 142), CFKPLAEIDSIECSYGIK (SEQ ID NO: 143), KPLAEDISIELSYGIK (SEQ ID NO: 145), KPLAEIGDIELSYGIK (SEQ ID NO: 146), KPLAEGDIELSYGIK (SEQ ID NO: 147), KPLAEIELSYGIK (SEQ ID NO: 148), KPLAEIDSIELTYGIK (SEQ ID NO: 149), KPLAEIDGIELSYGIK (SEQ ID NO: 150), KPLAEIDGIELTYGIK (SEQ ID NO: 151), KPLAEIGSIELSYGIK (SEQ ID NO: 152), KGLAEIDSIELSYGIK (SEQ ID NO: 153), KPLAGIDSIGLSYGIK (SEQ ID NO: 154), KCLAEIDSCELSYGIK (SEQ ID NO: 155) und CFKPLAEIDSIEC (SEQ ID NO: 156) oder eine Variante oder ein Fragment davon;
vi) eine Aminosäuresequenz ausgewählt aus der Gruppe bestehend aus LAEIDSIELSYGIK (SEQ ID NO: 170), AEIDSIELSYGIK (SEQ ID NO: 171), EIDSIELSYGIK (SEQ ID NO: 172), IDSIELSYGIK (SEQ ID NO: 173), DSIELSYGIK (SEQ ID NO: 174), SIELSYGIK (SEQ ID NO: 175), IELSYGIK(SEQ ID NO: 176) oder eine Variante oder ein Fragment davon; oder
vii) eine Aminosäuresequenz ausgewählt aus der Gruppe bestehend aus KPLAEIDSIELSYGI (SEQ ID NO: 179), KPLAEIDSIELSYG (SEQ ID NO: 180), KPLAEIDSIELSY (SEQ ID NO: 181), KPLAEIDSIELS (SEQ ID NO: 182), KPLAEIDSIEL (SEQ ID NO: 183), KPLAEIDSIE (SEQ ID NO: 184) oder eine Variante oder ein Fragment davon;
oder wobei das Peptid oder das Peptidderivat ausgewählt ist aus der Gruppe bestehend aus GHK (SEQ ID NO: 188), Oxytocin (SEQ ID NO: 186), Polymyxin B, LL37 (SEQ ID NO: 187), FOL-199 (SEQ ID NO: 185) und Becaplermin;
oder wobei das Peptid oder das Peptidderivat ein antimikrobielles Peptid oder Peptidderivat oder ein entzündungshemmendes Peptid oder Peptidderivat ist.

11. Zusammensetzung nach einem der vorhergehenden Ansprüche, wobei das Peptidderivat ein Peptid nach einem der vorhergehenden Ansprüche ist, welches an eine Einheit konjugiert ist, wie z. B. eine Einheit ausgewählt aus der Gruppe bestehend aus Polyethylenglykol (PEG), Monosacchariden, Fluorophoren, Chromophoren, radioaktiven Verbindungen und zellpenetrierenden Peptiden; und/oder wobei das Peptidderivat ein Peptid nach einem der vorhergehenden Ansprüche ist, welches durch Glykosylierung oder durch PEGylierung, Amidierung, Veresterung, Acylierung, Acetylierung und/oder Alkylierung modifiziert ist; und/oder wobei das Peptidderivat ein Peptid nach einem der vorhergehenden Ansprüche ist, welches mit einem anderen Polypeptid, wie z. B. einem Polypeptid ausgewählt aus der Gruppe bestehend aus Glutathion-S-Transferase (GST) und Protein A, oder mit einem Tag verschmolzen ist.

12. Zusammensetzung nach einem der vorhergehenden Ansprüche, wobei die Zusammensetzung in Form einer Salbe, eines Pulvers, eines Sprays, einer Lotion, eines Gels, eines Schaums, einer Creme, eines Make-ups oder eines Shampoos vorliegt.

13. Zusammensetzung nach einem der vorhergehenden Ansprüche zur Verwendung als ein Medikament.

14. Zusammensetzung nach einem der vorhergehenden Ansprüche zur Verwendung bei der Behandlung oder Prävention einer Krankheit oder einer Erkrankung im Zusammenhang mit Haarverlust oder einer dermatologischen Erkrankung, wie z. B. einer dermatologischen Erkrankung ausgewählt aus der Gruppe bestehend aus Psoriasis, atopischer Dermatitis, Ekzem, präkarzinogenen Zuständen und Hautinfektionen.

15. Verfahren zur Herstellung der Zusammensetzung nach einem der Ansprüche 1 bis 12, welches folgende Schritte umfasst:
a) Mischen des Peptids mit einem Saccharid;
b) Gefriertrocknen der Mischung von a);
c) Mischen von b) mit einem Lipid und einem Tensid;
d) Mahlen der Mischung von c); und
e) wahlweise Mischen der Mischung von d) mit einem Lipid und einem Verdickungsmittel.

## Revendications

1. Composition comprenant :
i) 0,01 à 2 % en poids de peptide ou dérivé de peptide ;
ii) 0,01 à 4 % en poids de saccharide ou saccharide modifié ;
iii) 35 à 50 % en poids de pétrolatum ; et
iv) 40 à 60 % en poids de myristate d'isopropyle,
à condition que la somme des composants de la composition totale ne dépasse pas 100 % en poids.

2. Composition selon la revendication 1, dans laquelle la composition comprend en outre 1 à 8 % en poids d'épaississant, tel que le béhénate de glycéryle ou la cire de carnauba.

3. Composition selon l'une quelconque des revendications précédentes, dans laquelle la composition comprend en outre 2 à 10 % en poids de tensioactif, tel que le laurate de sorbitan.

4. Composition selon l'une quelconque des revendications précédentes, dans laquelle le saccharide ou le saccharide modifié est choisi dans le groupe constitué du saccharose, du mannitol et du glucose.

5. Composition selon l'une quelconque des revendications précédentes, dans laquelle la composition comprend ou est constituée de :
a. 0,01 à 2 % en poids de peptide ou dérivé de peptide ;
b. 0,01 à 4 % en poids de saccharose, de mannitol ou de glucose ;
c. 1 à 8 % en poids de béhénate de glycéryle ou de cire de carnauba ;
d. 35 à 45 % en poids de pétrolatum ;
e. 40 à 60 % en poids de myristate d'isopropyle ; et
f. 2 à 10 % en poids de tensioactif, tel que le laurate de sorbitan.

6. Composition selon l'une quelconque des revendications précédentes, dans laquelle la composition comprend ou est constituée de :
i) 0,01 à 2 % en poids de peptide ou dérivé de peptide ;
ii) 0,01 à 4 % en poids de saccharose ;
iii) 3 % en poids de béhénate de glycéryle ;
iv) 42,4 % en poids de pétrolatum ;
v) 50 % en poids de myristate d'isopropyle ; et
vi) 4 % en poids de laurate de sorbitan,
tel que dans laquelle la composition comprend ou est constituée de :
a. 0,2 % en poids de peptide ou dérivé de peptide ;
b. 0,4 % en poids de saccharose ;
c. 3 % en poids de béhénate de glycéryle ;
d. 42,4 % en poids de pétrolatum ;
e. 50 % en poids de myristate d'isopropyle ; et
f. 4 % en poids de laurate de sorbitan.

7. Composition selon l'une quelconque des revendications précédentes, dans laquelle la composition comprend des particules comprenant ou constituées du peptide ou dérivé de peptide et du saccharide ou saccharide modifié, dans laquelle au moins 50 % des particules ont un diamètre moyen de particules compris entre 0,1 et 50 pm, par exemple entre 0,1 et 15 pm, tel qu'entre 0,1 et 10 pm, tel qu'entre 0,1 et 2 pm.

8. Composition selon l'une quelconque des revendications précédentes, dans laquelle le peptide ou dérivé de peptide comprend ou est constitué de 3 à 50 résidus d'acides aminés, tels que de 3 à 40 résidus d'acides aminés, tels que 5 à 30 résidus d'acides aminés, tels que 10 à 25 résidus d'acides aminés.

9. Composition selon l'une quelconque des revendications précédentes, dans laquelle le peptide ou dérivé de peptide comprend :
i) une séquence d'acides aminés de formule générale :
VDTYDGZ₇Z₈SVVYGLR (SEQ ID NO: 165) dans laquelle :
Z₇ est D ou G ;
Z₈ est I ou G ;
ii) une séquence d'acides aminés de formule générale :
KX₂LAX₅X₆X₇X₈IX₁₀LX₁₂YGIK (SEQ ID NO: 140) dans laquelle :
X₂ est C, P ou G ;
X₅ est E ou G ;
X₆ est C, D ou I ;
X₇ est D, I, S ou G ;
X₈ est S, D ou G ;
X₁₀ est E ou G ;
X₁₂ est S ou T ;
à condition que si X₁₂ est T, le peptide ne comprenne pas plus de 25 résidus d'acides aminés ; et
à condition que si X₂ est P, X₅ est E, X₆ est I, X₇ est D, X₈ est S, X₁₀ est E et X₁₂ est S, le peptide ne comprenne pas plus de 85 résidus d'acides aminés ;
iii) une séquence d'acides aminés de formule générale :
X₁₀LX₁₂YGIK (SEQ ID NO: 177)
dans laquelle :
X₁₀ est E ou G ;
X1₂ est S ou T ;
à condition que si X₁₂ est T, le peptide ne comprenne pas plus de 25 acides aminés ; et
à condition que si X₁₀ est E et X₁₂ est S, le peptide ne comprenne pas plus de 85 résidus d'acides aminés ;
iv) une séquence d'acides aminés de formule générale :
VDVPZ₅GDISLAYZ₁₃LR (SEQ ID NO: 164)
dans laquelle :
Z₅ est E ou N ;
Z₁₃ est R ou G ;
v) une séquence d'acides aminés de formule générale :
VDTYDGZ₇Z₈SVVYGLR (SEQ ID NO: 165)
dans laquelle :
Z₇ est D ou G ;
Z₈ est I ou G ;
vi) une séquence d'acides aminés de formule générale :
GDPNZ₅Z₆Z₇Z₈Z₉SWYGLR (SEQ ID NO: 166)
dans laquelle :
Z₅ est D ou G ;
Z₆ est D ou G ;
Z₇ est I ou R ;
Z₈ est G ou absent ;
Z₉ est D ou absent ;
vii) une séquence d'acides aminés de formule générale :
KX₂LAX₅X₆X₇X₈IX₁₀LSYGIK (SEQ ID NO: 162)
dans laquelle :
X₂ est C, P ou G ;
X₅ est E ou G ;
X₆ est C, I ou absent ;
X₇ est D, G ou absent ;
X₈ est S, G ou absent ;
X₁₀ est E ou G ;
viii) une séquence d'acides aminés de formule générale :
KX₂LAX₅IX₁₀LSYGIK (SEQ ID NO: 163)
dans laquelle :
X₂ est C, P ou G ;
X₅ est E ou G ;
X₁₀ est E ou G ;
i) une séquence d'acides aminés de formule générale :
Z₇Z₈SZ₁₀Z₁₁YGLR (SEQ ID NO: 178)
dans laquelle :
Z₇ est D ou G ;
Z₈ est I ou G ;
Z₁₀ est V ou L ;
Z₁₁ est V ou A ;
ou
ix) une séquence d'acides aminés de formule générale :
VDZ₃Z₄Z₅GZ₇Z₈SZ₁₀Z₁₁YGLR (SEQ ID NO: 68)
dans laquelle :
Z₃ est T ou V ;
Z₄ est Y ou P ;
Z₅ est D ou N ;
Z₇ est D ou G ;
Z₈ est I ou G ;
Z₁₀ est V ou L ;
Z₁₁ est V ou A.

10. Composition selon la revendication 1, dans laquelle le peptide ou dérivé de peptide comprend ou est constitué de :
i) la séquence d'acides aminés de VDTYDGDISWYGLR (SEQ ID NO: 1 ; FOL-005) ou un fragment ou variant de celle-ci ;
ii) la séquence d'acides aminés de KPLAEIDSIELSYGIK (SEQ ID NO: 136, FOL-014) ou un fragment ou variant de celle-ci ;
iii) la séquence d'acides aminés de VDVPNGDISLAYGLR (SEQ ID NO: 69, FOL-004) ou un fragment ou variant de celle-ci ;
iv) une séquence d'acides aminés choisie dans le groupe constitué de GDPNDGRGDSVVYGLR (SEQ ID NO: 137), VDTYDGGISVVYGLR (SEQ ID NO: 138), VDTYDGDGSVVYGLR (SEQ ID NO: 139), VDVPEGDISLAYGLR (SEQ ID NO: 157), LDGLVRAYDNISPVG (SEQ ID NO: 158), GDPNGDISVVYGLR (SEQ ID NO: 159), VDVPNGDISLAYRLR (SEQ ID NO: 160), VDVPEGDISLAYRLR (SEQ ID NO: 161), V(beta-D)TYDGDISWYGLR (SEQ ID NO:167), VDTY(beta-D)GDISWYGLR (SEQ ID NO: 168) et VDTYDG(beta-D) ISVVYGLR (SEQ ID NO:169) ;
v) une séquence d'acides aminés choisie dans le groupe constitué de KCLAECDSIELSYGIK (SEQ ID NO: 141), CLAEIDSC (SEQ ID NO: 142), CFKPLAEIDSIECSYGIK (SEQ ID NO: 143), KPLAEDISIELSYGIK (SEQ ID NO: 145), KPLAEIGDIELSYGIK (SEQ ID NO: 146), KPLAEGDIELSYGIK (SEQ ID NO: 147), KPLAEIELSYGIK (SEQ ID NO: 148), KPLAEIDSIELTYGIK (SEQ ID NO: 149), KPLAEIDGIELSYGIK (SEQ ID NO: 150), KPLAEIDGIELTYGIK (SEQ ID NO: 151), KPLAEIGSIELSYGIK (SEQ ID NO: 152), KGLAEIDSIELSYGIK (SEQ ID NO: 153), KPLAGIDSIGLSYGIK (SEQ ID NO: 154), KCLAEIDSCELSYGIK (SEQ ID NO: 155) et CFKPLAEIDSIEC (SEQ ID NO: 156), ou un variant ou fragment de celles-ci ;
vi) une séquence d'acides aminés choisie dans le groupe constitué de LAEIDSIELSYGIK (SEQ ID NO: 170), AEIDSIELSYGIK (SEQ ID NO: 171), EIDSIELSYGIK (SEQ ID NO: 172), IDSIELSYGIK (SEQ ID NO: 173), DSIELSYGIK (SEQ ID NO: 174), SIELSYGIK (SEQ ID NO: 175), IELSYGIK(SEQ ID NO: 176), ou un variant ou fragment de celles-ci ; ou
vii) une séquence d'acides aminés choisie dans le groupe constitué de KPLAEIDSIELSYGI (SEQ ID NO: 179), KPLAEIDSIELSYG (SEQ ID NO: 180), KPLAEIDSIELSY (SEQ ID NO: 181), KPLAEIDSIELS (SEQ ID NO: 182), KPLAEIDSIEL (SEQ ID NO: 183), KPLAEIDSIE (SEQ ID NO: 184), ou un variant d'un fragment de celles-ci ;
ou dans laquelle le peptide ou dérivé de peptide est choisi dans le groupe constitué de GHK (SEQ ID NO: 188), l'oxytocine (SEQ ID NO: 186), la polymyxine B, LL37 (SEQ ID NO: 187), FOL-199 (SEQ ID NO: 185) et la bécaplermine ;
ou dans laquelle le peptide ou dérivé de peptide est un peptide ou dérivé de peptide antimicrobien ou un peptide ou dérivé de peptide anti-inflammatoire.

11. Composition selon l'une quelconque des revendications précédentes, dans laquelle le dérivé de peptide est un peptide selon l'une quelconque des revendications précédentes, qui est conjugué à un groupement, tel qu'un groupement choisi dans le groupe constitué du polyéthylène glycol (PEG), des monosaccharides, des fluorophores, des chromophores, des composés radioactifs et des peptides pénétrant dans les cellules ; et/ou dans laquelle le dérivé de peptide est un peptide selon l'une quelconque des revendications précédentes, qui est modifié en étant glycosylé ou par PEGylation, amidation, estérification, acylation, acétylation et/ou alkylation ; et/ou dans laquelle le dérivé de peptide est un peptide selon l'une quelconque des revendications précédentes, qui est fusionné à un autre polypeptide, tel qu'un polypeptide choisi dans le groupe constitué de la glutathione-S-transférase (GST) et la protéine A, ou à une étiquette.

12. Composition selon l'une quelconque des revendications précédentes, dans laquelle la composition se présente sous la forme d'une pommade, d'une poudre, d'un spray, d'une lotion, d'un gel, d'une mousse, d'une crème, d'un maquillage ou d'un shampooing.

13. Composition selon l'une quelconque des revendications précédentes pour une utilisation comme médicament.

14. Composition selon l'une quelconque des revendications précédentes pour une utilisation dans le traitement ou la prévention d'une maladie ou d'une pathologie associée à la chute des cheveux ou d'une pathologie dermatologique, telle qu'une pathologie dermatologique choisie dans le groupe constitué du psoriasis, de la dermatite atopique, de l'eczéma, des états précarcinogéniques et des infections cutanées.

15. Procédé de fabrication de la composition selon l'une quelconque des revendications 1 à 12 comprenant les étapes suivantes :
a) mélange du peptide avec un saccharide ;
b) lyophilisation du mélange de a) ;
c) mélange de b) avec un lipide et un tensioactif ;
d) broyage du mélange de c) ; et
e) éventuellement mélange du mélange de d) avec un lipide et un épaississant.
